(19) Europäisches Patentamt
European Patent Office
Office européen des brevets

(11) **EP 1 466 000 B1**

(12) **EUROPEAN PATENT SPECIFICATION**

(45) Date of publication and mention
of the grant of the patent:
**13.08.2008 Bulletin 2008/33**

(51) Int Cl.:
*C12N 15/67* *(2006.01)*

(21) Application number: **02777463.7**

(22) Date of filing: **01.11.2002**

(86) International application number:
**PCT/GB2002/004952**

(87) International publication number:
**WO 2003/038099 (08.05.2003 Gazette 2003/19)**

(54) **REGULATION OF GENE TRANSCRIPTION BY THE VARIABLE NUMBER OF TANDEM REPEATS (VNTR) DOMAIN OF THE DOPAMINE TRANSPORTER GENE**

REGULIERUNG DER GENTRANSKRIPTION DURCH DIE VNTR DOMÄNE DES DOPAMIN TRANSPORTER GENS

REGULATION DE L'EXPRESSION GENIQUE PAR LE DOMAINE VNTR DU GENE CODANT POUR LE TRANSPORTEUR DE LA DOPAMINE

(84) Designated Contracting States:
**AT BE BG CH CY CZ DE DK EE ES FI FR GB GR IE IT LI LU MC NL PT SE SK TR**

(30) Priority: **01.11.2001 GB 0126268**

(43) Date of publication of application:
**13.10.2004 Bulletin 2004/42**

(73) Proprietor: **TCS CellWorks Ltd**
**Liverpool L2 4XE (GB)**

(72) Inventors:
• **QUINN, John**
**Hooton,**
**Cheshire CH62 9ES (GB)**
• **CARTWRIGHT, Terence**
**Aylesbury,**
**Bucks HP 22 4JA (GB)**

(74) Representative: **Hill, Christopher Michael et al**
**Page White & Farrer**
**Bedford House**
**John Street**
**London, WC1N 2BF (GB)**

(56) References cited:
• FISKERSTRAND C E ET AL: "An intronic polymorphic domain often associated with susceptibility to affective disorders has allele dependent differential enhancer activity in embryonic stem cells." FEBS LETTERS, vol. 458, no. 2, pages 171-174, XP002240215 ISSN: 0014-5793 cited in the application
• MACKENZIE ALASDAIR ET AL: "A serotonin transporter gene intron 2 polymorphic region, correlated with affective disorders, has allele-dependent differential enhancer-like properties in the mouse embryo." PROCEEDINGS OF THE NATIONAL ACADEMY OF SCIENCES OF THE UNITED STATES, vol. 96, no. 26, 21 December 1999 (1999-12-21), pages 15251-15255, XP002240216 Dec. 21, 1999 ISSN: 0027-8424 cited in the application
• FUKE S ET AL: "The VNTR polymorphism of the human dopamine transporter (DAT1) gene affects gene expression." PHARMACOGENOMICS JOURNAL, vol. 1, no. 2, 2001, pages 152-156, XP001147910 ISSN: 1470-269X
• SABOL SUE Z ET AL: "A functional polymorphism in the monoamine oxidase A gene promoter." HUMAN GENETICS, vol. 103, no. 3, 1998, pages 273-279, XP002240218 ISSN: 0340-6717
• PAQUETTE JEAN ET AL: "The INS 5' variable number of tandem repeats is associated with IGF2 expression in humans." JOURNAL OF BIOLOGICAL CHEMISTRY, vol. 273, no. 23, 5 June 1998 (1998-06-05), pages 14158-14164, XP002240219 ISSN: 0021-9258
• CHEVALIER DANY ET AL: "Characterization of new mutations in the coding sequence and 5'-untranslated region of the human prostacyclin synthase gene (CYP8A1)." HUMAN GENETICS, vol. 108, no. 2, February 2001 (2001-02), pages 148-155, XP002240220 ISSN: 0340-6717

- **VANDENBERGH D J ET AL: "HUMAN DOPAMINE TRANSPORTER CDNA PREDICTS REDUCED GLYCOSYLATION, DISPLAYS A NOVEL REPETITIVE ELEMENT AND PROVIDES RACIALLY-DIMORPHICTAQI RFLPS" MOLECULAR BRAIN RESEARCH, ELSEVIER SCIENCE BV, AMSTERDAM, NL, vol. 15, no. 1/2, September 1992 (1992-09), pages 161-166, XP000406137 ISSN: 0169-328X**
- **NAKAMURA YUSUKE ET AL: "VNTR (variable number of tandem repeat) sequences as transcriptional, translational, or functional regulators." JOURNAL OF HUMAN GENETICS, vol. 43, no. 3, 1998, pages 149-152, XP002240221 ISSN: 1434-5161 cited in the application**
- **MICHELHAUGH SHARON K ET AL: "The dopamine transporter gene (SLC6A3) variable number of tandem repeats domain enhances transcription in dopamine neurons." JOURNAL OF NEUROCHEMISTRY, vol. 79, no. 5, December 2001 (2001-12), pages 1033-1038, XP002240222 ISSN: 0022-3042**

**Description**

The present invention relates to a method for identifying compounds capable of regulating the expression of a target gene. The invention also relates to expression vectors, and to cell lines and kits comprising expression vectors, which are useful in such a method. In another aspect, the invention relates to a method for regulating the expression of a gene in a cell. The invention also relates to uses of such expression vectors.

The regulation of gene expression is an important therapeutic target in medicine. A number of effective pharmaceutical compounds are known to exert their beneficial effects by enhancing or suppressing the transcription of particular genes. Particular Examples include steroidal drugs and antagonists of endogenous steroids, such as hydrocortisone and spironolactone. An increase or decrease in transcription of an mRNA often results in a corresponding change in the production of the particular protein which is translated from the RNA. Therefore the regulation of transcription is an important way of controlling the level of a therapeutically-important protein within a cell.

It is often desirable in the treatment of disease to specifically target a particular protein present in the body, in order to enhance, suppress or modify its function. The protein may be targeted by the identification of compounds which bind selectively to the protein, or by regulating the expression of the protein. A problem with therapeutic methods which involve compounds binding directly to the protein is that it is difficult to identify compounds which bind only to the particular target protein. Almost all clinically-important drugs in use today bind to proteins other than their intended target to some extent. They are therefore only selective for their target protein rather than truly specific. Binding of drugs to non-target sites often results in unwanted side-effects, particularly as the therapeutic dose is increased, limiting the usefulness of the drug and the therapeutic dose which can be applied.

Methods which involve the regulation of gene expression hold the promise of avoiding some of the disadvantages of the above methods. Because the gene encoding for each protein has a unique nucleotide sequence, compounds may be identified which specifically modulate transcription of the gene of interest, thereby controlling the level of expression of the protein. Such compounds may act directly by binding to DNA or may act through modulating the action of transcription factors. Whereas the three-dimensional geometry of proteins is highly variable and difficult to predict, specific targeting of particular DNA sequences may be more readily achieved with a knowledge of the nucleotide sequence. Furthermore, the regulation of the transcription of a particular gene may result in a relatively long-lasting change in protein levels and thus protein function, compared to methods where protein function is only modulated by direct action of a drug at the protein itself.

Nevertheless, methods involving the regulation of gene expression may also suffer from problems of lack of specificity. Anti-sense oligonucleotides can usually be designed which bind specifically to a unique sequence in a gene, often thereby blocking translation of a particular transcribed mRNA, but the use of polynucleotides as therapeutic agents suffers from other drawbacks and can only be used to reduce rather than enhance protein production. An alternative strategy is to regulate gene expression by the use of a compound which binds to a polynucleotide sequence involved in the regulation of transcription. Suitable regulatory sequences are typically those which bind transcription factors *in vivo*, and so compounds which are structurally related to such transcription factors or which modulate the activity these transcription factors may be useful in regulating gene expression.

However, a problem with such strategies is that despite the uniqueness of each gene sequence, many sequence regulatory elements are shared between genes. Thus a single sequence regulatory element may be found in a number of different genes, and a single transcription factor may modulate the transcription of multiple genes. There is therefore a problem of achieving specificity in the regulation of a single gene using this method.

A number of more specific sequence regulatory elements exist, but their application is inevitably confined to the specific genes in which they are found. For instance, recent studies have demonstrated that a Variable Number Tandem Repeat (VNTR) sequence in the human serotonin transporter gene (SERT) can function as an enhancer of gene transcription and, furthermore, that allelic variants of the hSERT VNTR exhibit quantitatively and qualitatively different transcriptional activities (Fiskerstrand, C. E. et al., 1999; MacKenzie, A. and Quinn, J., 1999).

Fiskerstrand, C.E. *et al*, 1999 discloses a vector comprising 10 or 12 repeats of human serotonin transporter gene intron 2 variable number of repeats (VNTR) domain cloned in 5' of a luciferase gene.

MacKenzie, A. and Quinn, J., 1999 discloses a similar vector with a beta-galactosidase gene.

Fuke, S. et al, Pharmacogenomics Journal, 2001, 1, pages 152-156 describes the determination of the genomic structure of DAT1 genes containing 7, 9, 10 and 11 repeat alleles and examines the effect of the VNTR polymorphism in the 3'-UTR region of DAT1 on gene expression using the luciferase reporter system in COS-7 cells

In methods involving the regulation of gene expression, there is also the problem of variation in the sequence of regulatory elements between individuals within the population. Such sequence variations may influence the ability of compounds to regulate transcription through the regulatory element. Thus the effectiveness of such compounds may vary between individuals in the population.

There is therefore a need for new, more selective methods of regulating the expression of therapeutically-significant genes. In particular, there is a need for compounds which are capable of selectively regulating the expression of particular genes. In order to achieve this objective, new methods and tools for use in the identification of selective transcription-regulatory compounds are required. There is also a need for new methods and tools for identifying compounds that are capable of differentially regulating the expression of different alleles of a particular gene.

The present invention aims to facilitate the achievement of the above objectives. Accordingly the present invention provides an expression vector comprising:

(a) an enhancer sequence; and
(b) a reporter gene;

wherein the enhancer sequence comprises:

(i) the sequence of SEQ ID NO:1; or
(ii) a sequence of at least 10 nucleotides which shows at least 80% sequence identity with a part of SEQ ID NO:1

and wherein the enhancer sequence is capable of promoting the transcription of the reporter gene.

In this invention, SEQ ID NO:1 is a DNA molecule having the following sequence.
5'-CGTGTACTACCCCAGGACGCATGCAGGGCCCCCACTGGA G-3'

In a further aspect, the present invention provides a kit comprising two or more expression vectors, wherein each expression vector independently comprises

(a) an enhancer sequence; and
(b) a reporter gene;

wherein the enhancer sequence comprises:

(i) the sequence of SEQ ID NO:1; or
(ii) a sequence of at least 10 nucleotides which shows at least 80% sequence identity with a part of SEQ ID NO:1;

and wherein the enhancer sequence is capable of promoting the transcription of the reporter gene.

In a further aspect, the present invention provides a cell comprising an expression vector as defined above.

In a further aspect, the present invention provides method for identifying a compound capable of regulating the expression of a target gene, wherein the target gene comprises an enhancer sequence as defined above, which method comprises:

(a) providing an expression vector as defined above;
(b) introducing the expression vector into a cell;
(c) contacting the cell with a compound; and
(d) detecting the expression of the reporter gene.

A further aspect not according to the present invention is a compound identified by a method as defined above.

A further aspect not according to the present invention is a method for regulating the expression of a target gene in a cell, wherein the gene comprises an enhancer sequence as defined above, which method comprises:

(a) identifying a compound capable of regulating the expression of the target gene by a method as defined above; and

(b) contacting the cell with the compound.

In a further aspect not according to the present invention, a process for the manufacture of a medicament for regulating expression of a target gene is provided, wherein the target gene comprises an enhancer sequence as defined above, which process comprises identifying a compound capable of regulating the expression of the target gene by a method as defined in above, and using the compound as an active ingredient in the medicament

In a further aspect, the present invention provides use of an expression vector for identifying a compound capable of regulating the expression of a target gene, wherein the expression vector and the target gene each independently comprise an enhancer sequence as defined above.

In a further aspect not according to the present invention is the use of a compound for regulating the expression of a target gene, wherein the target gene comprises an enhancer sequence as defined above and the compound is capable of regulating the expression of a reporter gene comprised in an expression vector, which expression vector further comprises an enhancer sequence as defined above.

The expression vectors of the present invention are useful tools for identifying compounds which are capable of regulating the expression of genes. In particular, the present vectors advantageously facilitate the identification of compounds which selectively regulate the expression of particular genes comprising an enhancer sequence as defined above.

The kits of the present invention, comprising two or more expression constructs, are useful in particular for identifying compounds which differentially regulate the expression of genes comprising variant enhancer sequences. Such kits can advantageously facilitate the identification of compounds that differentially regulate the expression of different alleles of a gene, in a preferred embodiment of the invention.

The invention will now be described in more detail with reference to the following figures:

Figure 1A shows an alignment of a representative DAT VNTR and a representative SERT VNTR. Each sequence corresponds to repeat 3 of the VNTR sequence. The highlighted regions show the regions of maximum homology.

Figure 1B shows the results of an electrophoretic mobility shift assay using radiolabelled SERT VNTR (repeat number 3) as a probe. The radiolabelled probe was mixed with protein derived from ES cells and 10ng, 50ng and 100ng of unlabelled competitor DNA (SERT VNTR, DAT VNTR and a non-specific oligonucleotide). After incubation, the mixture was separated on a non-denaturing gel, the gel was dried and autoradiographed. The results show that the DAT VNTR sequence (but not the non-specific oligonucleotide) is capable of binding the protein that binds to the SERT VNTR sequence.

Figure 1C shows the results of an electrophoretic mobility shift assay using radiolabelled DAT VNTR (repeat number 3) as a probe. The radiolabelled probe was mixed with protein derived from ES cells and 10ng, 50ng and 100ng of unlabelled competitor DNA (SERT VNTR, DAT VNTR and a non-specific oligonucleotide). After incubation, the mixture was separated on a non-denaturing gel, the gel was dried and autoradiographed. The results show that the neither competitor is capable of binding the protein that binds to the DAT VNTR sequence.

Figure 2 shows SN4741 cells transfected with a plasmid containing the green fluorescence protein under the control of the hDAT VNTR enhancer, 24 h after transfection. The transfected cells are those showing green fluorescence.

Figure 3A shows an organotypic midbrain slice culture that has been transfected with a plasmid containing the green fluorescence protein under the control of the hDAT VNTR enhancer. Transfected cells exhibit green fluorescence.

Figure 3B shows an organotypic midbrain slice culture that has been transfected with a plasmid containing the green fluorescence protein under the control of the hDAT VNTR enhancer. The tissue slice has been stained with a monoclonal tyrosine hydroxylase antibody.

Figure 3C shows Figures 3A and 3B merged. Transfected cells that are dopaminergic fluoresce yellow.

Figure 3D shows an organotypic midbrain slice culture that has been transfected with a plasmid containing the green fluorescence protein under the control of the hDAT VNTR enhance. The arrow shows the gold bead that transfected the neuron in the field of view.

Figure 3E shows an organotypic midbrain slice culture that has been transfected with a control plasmid. No GFP is expressed by these cells.

Figure 3F shows an organotypic midbrain slice culture that has been transfected with a control plasmid. The arrow shows the gold bead that transfected the neuron in the field of view.

Figure 4 shows the GFP fluorescence generated in SN4741 cells transfected with pGE3P (a control plasmid comprising the GFP reporter gene under the control of an SV40 promoter) and in SN4741 cells transfected with pGE3P cells containing the DAT VNTR enhancer. It can be seen that expression from the GFP gene is increased in the presence of the DAT VNTR enhancer.

Figure 5 shows the luminescence generated in JAR cells transfected with pGL3 (a control plasmid comprising the luciferase reporter gene under the control of an SV40 promoter and a strong enhancer), in JAR cells transfected with a pGL3P plasmid (containing a promoter and the HD VNTR enhancer), and in JAR cells transfected with pGL3P (an enhancer-less vector). It can be seen that luciferase expression from the plasmide containing the HD VNTR enhancer is higher than luciferase expression from pGL3P.

**Expression vectors**

The expression vectors (also referred to as expression constructs) of the present invention are not particularly limited, provided that they contain an enhancer sequence and reporter gene as defined above. A wide range of suitable expression vector types will be well known to the skilled person. These include commercially available expression vectors designed for general recombinant procedures, for example plasmids that contain one or more reporter genes and regulatory elements required for expression of the reporter gene in cells. Suitable expression vectors include any plasmid, cosmid or phage construct that is capable of supporting expression of encoded genes in mammalian cells, such as pUC or Bluescript™ plasmid series. Alternatively, these vectors may be more complex, such as viral vectors discussed below.

Typically the regulatory elements of an expression vector will comprise a promoter sequence, which may be a weak promoter in the context of this invention in order to maximise the transcription-regulatory effect of the enhancer sequence. Preferably the promoter will only produce a low level of reporter gene expression in the absence of an enhancer sequence. The expression vector will also comprise a reporter gene, and also may include structures that assist in replication, such as origins of replication. In addition, the expression vector preferably contains multipurpose cloning regions that have numerous restriction enzyme sites. In the context of this invention, the cloning site for the enhancer sequence is preferably positioned appropriately to allow the transcription regulatory activity of the enhancer sequence to affect the reporter gene.

Vectors may also include a polyadenylation signal to effect proper polyadenylation of the transcript. Examples include SV40 and bovine growth hormone poly-A sites. In a preferred embodiment the expression construct comprises a terminator element. These elements can serve to enhance transcript levels and to minimize read through from the construct into other plasmid sequences. Finally, the expression vectors preferably comprise one or more selectable markers, often in the form of antibiotic resistance genes, that permit selection of cells that carry these vectors.

As stated above, in certain embodiments of the present invention, the expression construct comprises a virus or engineered construct derived from a viral genome. The ability of certain viruses to enter cells via receptor-mediated endocytosis and, in some cases, integrate into the host cell chromosomes, have made them attractive candidates for gene transfer in to mammalian cells.

### i. Retroviruses

Retroviruses are a group of single-stranded RNA viruses characterized by an ability to convert their RNA to double-stranded DNA in infected cells by a process of reverse-transcription. The resulting DNA can then stably integrate into cellular chromosomes as a provirus and direct synthesis of viral proteins. The integration results in the retention of the viral gene sequences in the recipient cell and its descendants. A typical retroviral genome contains three genes, gag, pol, and env, that code for capsid proteins, reverse transcriptase and integrase enzymes, and envelope proteins, respectively. The gag-encoded capsid proteins are responsible for assembling the virus particles and packaging the genomic RNA of the virus. The gag polyproteins contain a zinc-finger nucleic acid-binding domain. RNA sequences for packaging ("packing signals") in the virus are necessary for the virus to be packaged and experiments have shown that all or part of the packaging signal in viral RNA is located near the 5 end of the genome. These signals appear to be quite large, i.e., hundreds of nucleotides. Two long terminal repeat (LTR) sequences are present at the 5' and 3' ends of the viral genome. These LTRs contain strong promoter and enhancer sequences that are also required for integration into the host cell genome.

In one embodiment a retroviral vector is constructed by inserting an enhancer sequence operatively linked to a reporter gene into the proviral genome, which has been cloned into a plasmid, in the place of certain viral sequences to produce a virus that is replication-defective. In order to produce virions, a packaging cell line containing the gag, pol and env genes but without the LTR and packing signal components is constructed. When the recombinant plasmid containing the desired enhancer sequence linked to a reporter gene, together with the retroviral LTR and packing signal sequences is introduced into this packaging cell line (by calcium phosphate precipitation for example), the packing signal sequence allows the RNA transcript of the recombinant plasmid to be packaged into viral particles, which are then secreted into the culture medium. The medium containing the recombinant retroviruses is then collected, optionally concentrated, and used for gene transfer. Retroviral vectors are able to infect a broad variety of cell types. However, integration and stable expression of retrovirally packaged genes require the division of host cells. This may not occur in certain cell lines contemplated for use in this invention, particularly primary cell cultures and so retroviral vectors may not be appropriate for these sorts of cell lines. (for reviews see for example: Kim S.H. et al., Adv Virus Res 55: 545-546, "Retroviral vectors." 2000; Kurian K.M. et al., Mol Pathol 53(4): 173-173, "Retroviral vectors." 2000)

### ii. Adenoviruses and Adenoassociated viruses:

Human adenoviruses are double-stranded DNA tumor viruses with genome sizes of approximate 36 kB. Adenoviruses have well characterized as a model system for eukaryotic gene expression, which has made them an attractive system for development of a gene transfer system. This group of viruses is easy to grow and manipulate, and exhibit a broad host range in vitro and in vivo. In lytically infected cells, adenoviruses are capable of shutting off host protein synthesis, directing cellular machinery to synthesize large quantities of viral proteins, and producing copious amounts of virus.

The E1 region of the genome includes E1A and E1B which encode proteins responsible for transcription regulation of the viral genome, as well as a few cellular genes. E2 expression, including E2A and E2B, allows synthesis of viral replicative functions, e.g. DNA-binding protein, DNA polymerase, and a terminal protein that primes replication. E3 gene products prevent cytolysis by cytotoxic T cells and tumor necrosis factor and appear to be important for viral propagation. Functions associated with the E4 proteins include DNA replication, late gene expression, and host cell shutoff. The late gene products include most of the virion capsid proteins, and these are expressed only after most of the processing of a single primary transcript from the major late promoter has occurred. The major late promoter (MLP) exhibits high efficiency during the late phase of the infection.

As only a small portion of the viral genome appears to be required for the virus to be packaged, adenovirus-derived vectors offer excellent potential for the substitution of large DNA fragments when used in connection with packaging cell lines that express the essential viral proteins endogenously. In general, adenovirus gene transfer systems are based upon recombinant, engineered adenovirus which is rendered replication-incompetent by deletion of a portion of its genome, such as E1, and yet still retains its competency for infection. Sequences encoding relatively large foreign proteins can be expressed when additional deletions are made in the adenovirus genome. For example, adenoviruses deleted in both E1 and E3 regions are capable of carrying up to 10 kB of foreign DNA and can be grown to high titers in 293 cells. Persistent expression of transgenes following adenoviral infection has also been reported. (see for example Benihoud K., Yeh P. & Perricaudet M., Curr Opin Biotechnol 10(5): 440-440, "Adenovirus vectors for gene delivery." 1999; Ridoux V. et al., Neurobiol Dis 2(1): 49-45, "Adenovirus mediated gene transfer in organotypic brain slices." 1995)

Adeno-associated virus (AAV) is a defective, non-pathogenic human parvovirus that depends for growth on coinfection with a helper adenovirus or herpes virus. The wild-type virus can site specifically integrate at a single location on chromosome 19. Recombinant adeno-associated virus can also efficiently integrate into the host genome. Recombinant adeno-associated viruses (rAAVs) have been quite widely used for transgenesis (Kremer E.J. & Perricaudet M., Br Med Bull 51(1): 31-34, "Adenovirus and adeno-associated virus mediated gene transfer." 1995). In contrast to other gene delivery systems, rAAVs lack all viral genes and show long-term gene expression in vivo without immune response or toxicity. This vector system can be used for long-lasting genetic modification. In addition, vectors for regulatable gene expression and vectors retargeted to different cells have been engineered. Specifically, the AAV system has been used to study gene expression in the nervous system and techniques are known in the art for the production of AAV vectors appropriate for use with this invention (for a review see Quinn J.P., Fiskerstrand C.E., Gerrard L., Mackenzie A. & Payne C., Neuropeptides 34: 292-302, "Molecular models to analyse preprotachykinin-A expression and function" 2000).

The adenoviruses and adenoassociated viruses may be used with cell lines and organotypic cultures in which no cell division is taking place.

_iii. Artificial Chromosomes_

Since the open reading frames comprising each exon of a eukaryotic gene can be separated by large non-coding introns and since the control sequences in eukaryotic genes can exert their effects over large distances and so do not need to be located in a close proximity to their associated open reading frames, many eukaryotic genes are dispersed over large stretches of DNA in the eukaryote's genome. This means that in order for expression constructs of genes to work as they do in their native context, very large fragments of the native context need to be included in the expression vector. Bacterial and Yeast Artificial chromosomes may be used to create large expression vectors, if desired. A plasmid will typically allow 40 kilobases of DNA to be cloned into them while a Yeast Artificial Chromosome (YAC) allows up to a million bases of DNA to be cloned. (for a review see Quinn J.P., Fiskerstrand C.E., Gerrard L., Mackenzie A. & Payne C., Neuropeptides 34: 292-302, "Molecular models to analyse preprotachykinin-A expression and function" 2000)

_iv. Vector Delivery Methods:_

In order to effect expression of reporter gene constructs, the expression vector must be delivered into a cell. As described above, one mechanism for delivery is via viral infection where the expression vector is encapsulated in an infectious viral particle.

Several non-viral methods for the transfer of expression vectors into cultured mammalian cells are also contemplated by the present invention. These include calcium phosphate precipitation, DEAE-dextran, electroporation, direct microinjection, DNA-loaded liposomes and lipofectamine-DNA complexes, cell sonication, gene bombardment using high velocity microprojectiles, polycations and receptor-mediated transfection.

Another embodiment of the invention for transferring a naked DNA expression vector into cells may involve particle bombardment, sometimes referred to as biolistic transfer. This method depends on the ability to accelerate DNA coated microprojectiles to a high velocity allowing them to pierce cell membranes and enter cells without killing them. Several devices for accelerating small particles have been developed. One such device relies on a high voltage discharge to generate an electrical current, which in turn provides the motive force. The microprojectiles used have consisted of biologically inert substances such as tungsten or gold beads. (see for example Furth P.A., Mol Biotechnol 7(2): 139 - 143, "Gene transfer by biolistic process." 1997)

In a further embodiment of the invention, the expression vector may be entrapped in a liposome. Liposomes are vesicular structures characterized by a phospholipid bilayer membrane and an inner aqueous medium. Multilamellar liposomes have multiple lipid layers separated by aqueous medium. Liposomes form spontaneously when phospholipids are suspended in an excess of aqueous solution. The lipid components undergo self-rearrangement before the formation of closed structures and entrap water and dissolved solutes between the lipid bilayers. For delivery of DNA, cationic lipids are preferred.

In certain embodiments of the invention, the liposome may be complexed with hemagglutinin, the membrane fusion protein of the influenza virus . This can facilitate fusion with the cell membrane and promote cell entry of liposome-encapsulated DNA (Schoen P. et al., Gene Ther 6(5): 823-823, "Gene transfer mediated by fusion protein hemagglutinin reconstituted in cationic lipid vesicles." 1999).

In other embodiments, DNA expression vectors can be complexed with the nuclear non-histone chromosomal protein HMG-1, which facilitates nuclear uptake of the DNA. This approach may be used in conjunction with other delivery techniques such as liposomes. (see for example Isaka Y. et al., Exp Nephrol 6(2): 144-147, "The HVJ liposome method." 1998)

Another mechanism for transferring expression vectors into cells is receptor-mediated delivery. This approach takes advantage of the selective uptake of macromolecules by receptor-mediated endocytosis in almost all eukaryotic cells. Because of the cell type-specific distribution of various receptors, the delivery can be highly specific. Receptor-mediated gene targeting vehicles generally consist of two components: a cell receptor-specific ligand and a DNA-binding agent. Several ligands have been used for receptor-mediated gene transfer. The most extensively characterized ligands are asialoorosomucoid (Wu G.Y. et al., J Biol Chem 266(22): 14338-14334, "Receptor-mediated gene delivery in vivo. Partial correction of genetic analbuminemia in Nagase rats." 1991) and transferrin (Wagner E., Ogris M. & Zauner W., Adv Drug Deliv Rev 30: 97-113, "Polylysine-based transfection systems utilizing receptor-mediated delivery." 1998).

## Reporter genes

A reporter gene is a gene which produces a readily identifiable and assayable product. The gene encoding beta-galactosidase (Promega), green fluorescent protein (Clontech, Palo Alto, Calif.) and firefly luciferase (Promega, Madison, Wis.) are all useful in the present invention as these genes produce easily assayable products that can be readily assayed using spectrophotometry, fluorescence detection or other photometric assays. One skilled in the art will however recognize other reporter genes which are applicable in the present invention. Examples of such reporter genes include, but are not limited to, chloramphenicol acetyl transferase (Promega), human growth hormone (Amersham Life Science, Arlington Heights, Ill.), alkaline phosphatase (Clontech) and beta-glucuronidase (Clontech).

The method of present invention is distinguished from other techniques for identifying chemical compounds that modulate expression of genes, as it is capable of specifically identifying agents which affect expression of genes regulated by enhancers comprising the sequence of SEQ ID NO:1 or a related sequence. These agents are identified by their capacity to modulate the activity of such an enhancer sequence. A decrease in the enhancer activity is measured by a correspondent decrease in production of the reporter gene's product. Increase in activity of the enhancer activity is measured by a correspondent increase in production of the reporter gene's product. Thus, decrease in the production of, for example, beta-galactosidase, indicates that enhancer activity is being suppressed by the compound being tested; an increase in the production of beta-galactosidase, is indicative of stimulation of the enhancer activity of the enhancer. The effect in production of the assaying product reflects the effect on expression of the gene that is normally regulated by the enhancer that would occur in a cell treated with the compound.

## Enhancer sequences

The enhancer sequences of the present invention are sequences which are capable of regulating the expression of a reporter gene in the expression construct. Typically the enhancer sequence promotes the transcription of the reporter gene and thereby increases the level of the assayable product.

The enhancer sequence comprises the sequence of SEQ ID NO:1 or a related sequence. SEQ ID NO:1 is found in the 3' non-coding region of the human dopamine transporter (DAT) gene. The present application is founded on the disclosure that this sequence surprisingly shows enhancer activity and is capable of promoting the transcription of a reporter gene.

Accordingly, the enhancer sequence preferably comprises two or more repeats of the sequence of SEQ ID NO:1 or a related sequence. In other words, the enhancer sequence preferably comprises a VNTR sequence. The enhancer sequence may comprise 2, 3, 4, 5, 6, 7, 8, 9, 10, 11, 12, 13 or more repeats. In a preferred embodiment, the enhancer sequence comprises from 3 to 13 repeats of SEQ ID NO:1. More preferably the enhancer sequence comprises 9 or 10 repeats of SEQ ID NO:1.

The present invention may employ enhancer sequences which are homologous to the sequence of SEQ ID NO:1, and the target genes of the present invention may be genes other than the human DAT gene. Preferably the target gene is a neuronal gene, which means a gene which is expressed in any type of neuron.

In certain naturally-occurring genes, enhancer sequences are found as part of a tandemly repeated sequence. Many of these tandemly repeated sequences show variations in the number of tandem repeats in different individuals and these sequences are referred to as Variable Number Tandem Repeat (VNTR) sequences. For instance, in the human DAT gene, the sequence of SEQ ID NO:1 is found within a VNTR polymorphism. Alleles of this 40 nucleotide VNTR sequence range form 3 to 13 repeats, with the 9-repeat and 10 repeat alleles being the most common.

Enhancer sequences comprising VNTR repeats related to SEQ ID NO:1 include the enhancer of the human seratonin transporter (SERT; GENBANK accession U79746, nucleotides 20-218), the enhancer of the human Huntington's disease gene (HD; GENBANK accession number Z68274, nucleotides 20480-20255), the enhancer of the human B2-bradykinin receptor (GENBANK accession X86163, nucleotides 1700-1910), the enhancer of the N- methyl-D-aspartate receptor (GENBANK accession Z32774, nucleotides 6704-7090), the enhancer of human Factor VIII (GENBANK accession JO2933, nucleotides 2380-2570), Herpes Simplex Virus-1 (GENBANK accession X14112, nucleotides 151500-151671), the mouse immunoglobulin heavy chain/c-myc fusion gene (GENBANK accession S79793, nucleotides 237-452) and the equivalent human fusion gene present in Burkitt's lymphoma. Other genes comprising enhancer sequences that contain VNTR repeats related to SEQ ID NO:1 include DRD4, IL-1Ra, cytostatin B, monoamine oxidase A, BHSV (GENBANK accession X766943) and the Ig switch (GENBANK identity MMIGHMY). The VNTR repeats of these enhancers may be utilised in the present invention.

In the method for identifying a compound capable of regulating the expression of a target gene of the present invention, the gene and the DNA expression vector each comprise an enhancer sequence. In a preferred embodiment, the target gene and the DNA expression vector each comprise the same enhancer sequence. In this embodiment, an expression vector is constructed which contains an enhancer sequence identical to that of the target gene. This embodiment advantageously facilitates the identification of compounds which selectively regulate the expression of a particular gene having a specific enhancer sequence. A particular enhancer sequence within the scope of the present invention may be found naturally within only a small number of genes, or uniquely within only a single gene. Moreover, the enhancer sequence present within a particular target gene may be found to vary within different individuals in the population. Screening for compounds which selectively regulate transcription via such enhancers therefore facilitates improved specificity in gene targeting.

Enhancer sequences according to the present invention are found in a number of naturally-occurring genes. Such genes are accordingly suitable as target genes according to the present invention Target genes according to the present invention therefore include the human Huntington's disease gene, the human B2-bradykinin receptor, the N-methyl-D-aspartate receptor, human Factor VIII, the mouse immunoglobulin heavy chain/c-myc fusion gene (and the equivalent human fusion gene present in Burkitt's lymphoma), DRD4, IL-1Ra, cytostatin B, monoamine oxidase A, BHSV and the Ig switch.

Other target genes and enhancer sequences for use in the present invention may be identified in various ways. In one embodiment, target genes and enhancer sequences are identified by comparing the sequence of SEQ ID NO:1 to other known nucleotide sequences. For example, suitable target genes may be identified by comparing the sequence of SEQ ID NO:1 (GENBANK accession no. AF321321) with the GENBANK database maintained by the National Center for Biotechnology Information (NCBI, Bethesda, MD, USA). Such analysis may be performed using a local sequence alignment algorithm such as BLAST (Altschul, S.F., Gish, W., Miller, W., Myers, E.W. & Lipman, D.J., J. Mol. Biol. 215: 403-410, "Basic local alignment search tool." 1990) available using the World Wide Web server maintained by the NCBI, or Smith/Waterman (Journal of Molecular Biology 147:195-197, 1981). BLAST allows nucleic acid and amino acid sequences to be compared with public repositories of biological sequences to identify sequences that are closely related. In order to identify VNTR sequences according to a preferred embodiment of the present invention, genome sequence databases such as GENBANK can be searched with the sequence of SEQ ID NO:1 to find genes which contain homologous sequences that are tandemly repeated. The corresponding VNTR regions may then be amplified by PCR or could be chemically synthesised if desired.

Suitable enhancer sequences according to the present invention preferably comprise a sequence of at least 10 nucleotides which shows at least 80% sequence identity with a part of SEQ ID NO:1. Suitable enhancer sequences may vary from the sequence of SEQ ID NO:1 in that one or more nucleotides are substitute into, deleted from or inserted into the sequence of SEQ ID NO:1, provided that the resultant sequence still shows enhancer activity (that is, it is still capable of promoting transcription of a reporter gene). The degree of sequence identity (or homology) may be determined by aligning a putative enhancer sequence with the sequence of SEQ ID NO:1 and determining the region of highest similarity. The putative enhancer sequence may show sequence identity to any part of SEQ ID NO:1, that is to any sequence of 10 or more nucleotides within SEQ ID NO:1. Such an alignment and determination of the level of sequence identity may be performed using a computer program such as the BLAST, Smith/Waterman or FASTA algorithms. The percentage sequence identity is equal to the number of matching bases as a proportion of the length of the aligned sequence. Thus, if any sequence of 10 nucleotides in the putative enhancer sequence contains 7 or more nucleotides which are identical to the nucleotides present in SEQ ID NO:1 at their equivalent positions, then the degree of sequence identity (or homology) is at least 70%.

More preferably the enhancer sequence comprises a sequence of at least 10 nucleotides which shows at least 80% or at least 90% sequence identity with a part of SEQ ID NO:1. Most preferably the enhancer sequence comprises a sequence of at least 15, at least 20, or at least 30 nucleotides which shows at least 70% or at least 90% sequence identity with a part of SEQ ID NO:1.

Alternatively, suitable enhancer sequences and target genes may be identified by isolating nucleotide sequences from DNA libraries that hybridise to the sequence of SEQ ID NO:1. As the enhancer sequences of the present invention are typically found in the non-coding regions of genes, and often within introns, genomic DNA libraries are preferably used. Preferably the complementary strand of an enhancer sequence is capable of hybridising to the sequence of SEQ ID NO:1 under low stringency conditions. More preferably, the complementary strand of the enhancer sequence is capable of hybridising to the sequence of SEQ ID NO:1 under high stringency conditions.

Stringency conditions may be controlled by varying the temperature and salt concentration during hybridisation or during the post-hybridisation washes, according to standard techniques described previously (Old & Primrose (1994), Principles of Gene Manipulation, Blackwell Science and Maniatis et al. (1992), Molecular Cloning, A Laboratory Manual, Cold Spring Harbor Laboratory, New York). Stringency washes may be performed under successively more stringent conditions (lower salt or higher temperature) to find progressively more closely related sequences.

For high stringency hybridisation conditions, the hybridisation temperature should be close to the melting temperature ($T_m$) of the probe. $T_m$ is defined as the temperature at which the probe and its target are 50% dissociated, and for oligonucleotide probes may be calculated according to the "Wallace rule" by the following formula:

$$T_m = 4 \times (\text{number of G:C base-pairs}) + 2 \times (\text{number of A:T base-pairs})$$

For high stringency hybridisation conditions, the hybridisation temperature should be within 5°C of $T_m$. Accordingly, for a 20-mer oligonucleotide probe with 50% G-C content, the $T_m$ is about 60°C and a high stringency hybridisation temperature would be 55°C. A low salt hybridisation and wash buffer, such as 0.2 X SSC or 1 X SSC is used for high stringency conditions.

For lower stringency conditions, the hybridisation temperature is 5°C to 25°C, 5°C to 15°C or 5°C to 10°C below $T_m$ and the buffer has a higher salt content. Thus suitable low stringency hybridisation conditions for a 20-mer oligonucleotide probe with 50% G-C content would be 50°C using a high salt hybridisation and wash buffer, such as 6 X SSC.

Chaotropes can also reduce the melting temperature of a sequence, while other reagents such as alkyl ammonium ions and formamide can normalise the effects of high G-C content. Magnesium ions also promote hybridisation efficiency by neutralising the negative charges on the DNA phosphodiester backbone. Thus a high concentration of magnesium ions will reduce the stringency of hybridisation.

Accordingly, cloning procedures may be used to isolate and identify target genes and enhancer sequences according to the present invention. The first step in a cloning procedure is the screening of an appropriate DNA library. The screening procedure may be based on the hybridization of oligonucleotide probes, designed to be complementary to a part or the whole of the sequence of SEQ ID NO:1. Preferably the probes are at least 10 nucleotides in length, more preferably 10 to 40 nucleotides and most preferably 15 to 30 nucleotides in length. The operation of such screening protocols are well known to those of skill in the art and are described in detail in the scientific literature. The cloned or synthetic enhancer sequences can then be ligated into an appropriate vector which contains the desired reporter gene.

Once a putative enhancer sequence (which shows homology to the sequence of SEQ ID NO:1 or the complementary strand of which hybridises to SEQ ID NO:1) has been identifiied, the transcription-regulatory activity of the enhancer sequence can be assessed by constructing an expression vector comprising the enhancer sequence and a reporter gene, as discussed above. Where the enhancer sequence promotes the transcription of the reporter gene, the enhancer sequence falls within the scope of the present invention.

## Preparation of DNA expression vectors

The expression vectors of the present invention can be constructed by cloning of a genomic DNA molecule containing a suitable enhancer sequence using standard molecular biology techniques known in the art. Alternatively, having knowledge of the sequence of the desired enhancer, the enhancer sequence may be synthesized chemically according to standard techniques.

Methods of designing primers are well known in the art and it will be possible for one of ordinary skill in the art to design other primers for the enhancer sequence of SEQ ID NO:1 and to design primers for other related enhancer sequences according to the present invention (Rychlik W., Spencer W.J. & Rhoads R.E., Nucleic Acids Res 18(21): 6409-6412, "Optimization of the annealing temperature for DNA amplification in vitro." 1990; Lowe T. et al., Nucleic Acids Res 18(7): 1757-1761, "A computer program for selection of oligonucleotide primers for polymerase chain reactions." 1990; Breslauer K.J. et al., Proc Natl Acad Sci U S A 83(11): 3746-3750, "Predicting DNA duplex stability from the base sequence." 1986; Rychlik W. & Rhoads R.E., Nucleic Acids Res 17(21): 8543-8551, "A computer program for choosing optimal oligonucleotides for filter hybridization, sequencing and in vitro amplification of DNA." 1989; Wetmur J.G., Crit Rev Biochem Mol Biol 26(3-4): 227-259, "DNA probes: applications of the principles of nucleic acid hybridization." 1991).

The enhancer sequence of the human dopamine transporter gene, comprising a VNTR repeat, is found in the 3' untranslated region of the human dopamine transporter gene. Variants of this VNTR have been found with repeat numbers ranging from three to eleven repeats. This VNTR may be amplified using the following primers (Jacobsen L.K. et al., Am J Psychiatry 157: 1700-1703, "Prediction of dopamine transporter binding availability by genotype: a preliminary report." 2000):

5'-CTTCCTGGAGGTCACGGCTCAAGG-3' (SEQ ID NO:2)
5'-AGGAAATTCTGTTTATGTTCTTG-3' (SEQ ID NO:3)

In some situations it may be desirable to multimerise the VNTR sequences prior to cloning into an expression vector. Multimerisation will increase the effect of the enhancer sequence making the assay more sensitive. Multiple copies of the cloned VNTR sequence may be ligated together to generate multimerised sequences of different lengths, which can then cloned into expression vectors.

## Kits comprising expression vectors

The present invention also relates to kits comprising expression vectors. Such kits are useful for identifying compounds which differentially regulate transcription via similar but different enhancer sequences. In a preferred embodiment, the enhancer sequence of each expression vector in the kit comprises the sequence of a different allele of a gene.

Preferably the kit comprises expression vectors containing a VNTR enhancer sequence known to regulate a particular gene in the human genome, and each expression vector in the kit comprises a natural variant of the VNTR enhancer identified in the human population.

Target genes according to the present invention may show natural variation in the sequences of their enhancers. In particular, variations in the sequence of the VNTR enhancers of this invention can change the expression of their associated gene, and each VNTR enhancer may respond differently to compounds which interfere with this regulatory pathway. Some VNTR sequences may not even respond to certain compounds that affect other VNTR sequences. Variations in the sequence of the VNTR will alter the binding of the VNTR sequence to its associated transcription factors. The consequence of changes in the ability of the VNTR sequences to bind their associated transcription factor is a change in the regulatory properties of the VNTR, i.e. differences in sequence of the VNTR will directly alter the expression of their associated gene.

It is therefore desirable to be able to individually screen all of the natural variants of the enhancers of a particular target gene with libraries of compounds to find drug candidates that differentially modulate the behaviour of different natural variants of these VNTR enhancer sequences. In this way therapies can be tailored to an individual's genetic make-up.

In order to create a kit comprising expression vectors according to a particular embodiment of the present invention, the natural genetic variation in the VNTR sequences must be determined. This requires that the sequence of the VNTRs in different individuals must be determined. To ensure that substantially all of the natural variants of each VNTR in each gene is identified requires the analysis of a statistically large number of individuals, but screening for drugs with differential characteristics can still be performed as soon as two or more variants are identified.

Since the human genome is complete, the identification of tandemly repeated sequences can be achieved by searching the public databases for sequences that contain tandem repeats of the sequence of SEQ ID NO:1 or a related sequence. Once related tandemly repeated sequences have been identified, PCR primers can then be designed that bind to sequences that flank these tandemly repeated sequences using standard techniques known in the art. These can be used to selectively amplify the tandemly repeated sequences from DNA isolated from human subjects using polymerase chain reactions (PCR), which is a technique well known in the art. Comparison of the lengths of the amplification products from different individuals will show whether the repeated sequence has variable numbers of repeats and sequencing the amplification products will allow single nucleotide polymorphisms (SNPs) to be identified. PCR products that contain VNTR enhancers, which have different sequences can then be cloned into appropriate expression vectors using techniques known in the art.

**Cell lines comprising expression vectors**

In a further aspect, the present invention relates to a cell comprising an expression vector as defined above.

A host cell that has been transformed with an expression vector as described above may be used to screen for drugs that alter the regulatory properties of enhancers according to the present invention. A variety of host cell lines are applicable with this invention but the cell is preferably a mammalian cell and more preferably the cell is a neuronal cell. In a preferred embodiment, the enhancer sequence of the expression vector is identical to an enhancer sequence found in a gene expressed in neurons, and the cell line is a neuronal cell line. Neuronal cells and cell lines which can be utilized for transgenesis in the present invention include primary cell lines derived from living tissue such as rat or mouse brains and organotypic cell cultures, including brain slices from animals such as rats or mice. The PC12 cell line (available from the American Type Culture Collection, ATCC, Manassas, VA, USA) has been shown to express a number of neuronal marker proteins in response to Neuronal Growth Factor (NGF). The PC12 cell line is considered to be a neuronal cell line and is applicable for use with this invention. JAR cells (available from ATCC) are a platelet derived cell-line that express some neuronal genes, such as the serotonin transporter gene, and may be used with this invention.

WO 91/13150 describes a variety of cell lines, including neuronal cell lines, and methods of producing them. Similarly, WO 97/39117 describes a neuronal cell line and methods of producing such cell lines. The neuronal cell lines disclosed in these patent applications are applicable for use in the present invention.

Non-neuronal cell lines may be used in the present invention, including mouse embryonic stem cells. Cultured mouse embryonic stem cells can be used to analyse expression of genetic constructs using transient transfection with plasmid constructs. Mouse embryonic stem cells are pluripotent and undifferentiated. These cells can be maintained in this undifferentiated state by Leukaemia Inhibitory Factor (LIF).

Withdrawal of LIF induces differentiation of the embryonic stem cells. In culture, the stem cells form a variety of differentiated cell types. Differentiation is caused by the expression of tissue specific transcription factors, allowing the function of an enhancer sequence to be evaluated. (See for example Fiskerstrand C.E., Lovejoy E.A. & Quinn J.P., FEBS lett 458: 171-174, "An intronic polymorphic domain often associated with susceptibility to affective disorders has allele dependent differential enhancer activity in embryonic stem cells.") Yeast one-hybrid systems may also be used to identify compounds that inhibit specific protein/DNA interactions, when transcription factors for the VNTR enhancers have been identified.

Cell lines may be derived from a transgenic animal. Construction of a transgenic animal results in an organism that has an engineered construct present in all cells in the same genomic integration site. Thus cell lines derived from a transgenic animal will be consistent in as much as the engineered construct will be in the same genomic integration site in all cells and hence will suffer the same position effect variegation. In contrast, introducing genes into cell lines or primary cell cultures can give rise to heterologous expression of the construct. A disadvantage of this approach is that the expression of the introduced DNA may be affected by the specific genetic background of the host animal.

The expression vectors of this invention can be used to genetically modify mouse embryonic stem cells using techniques known in the art. Typically, the expression vector is introduced into cultured murine embryonic stem cells. Transformed ES cells are then injected into a blastocyst from a host mother and the host embryo re-implanted into the mother. This results in a chimeric mouse whose tissues are composed of cells derived from both the embryonic stem cells present in the cultured cell line and the embryonic stem cells present in the host embryo. Usually the mice from which the cultured ES cells used for transgenesis are derived are chosen to have a different coat colour from the host mouse into whose embryos the transformed cells are to be injected. Chimeric mice will then have a variegated coat colour. As long as the germ-line tissue is derived, at least in part, from the genetically modified cells, then the chimeric mice be crossed with an appropriate strain to produce offspring that will carry the transgene.

Primary tissue culture from these transgenic mice will then provide the necessary cell lines with the expression construct already integrated into the genome. (for an example see Mackenzie A. & Quinn J.P., Proc Natl Acad Sci USA 96: 15251-15255, "A serotonin transporter gene intron 2 polymorphic region, correlated with affective disorders, has allele-dependent differential enhancer-like properties in the mouse embryo" 1999).

**Method for identifying gene expression-regulatory compounds**

In a preferred embodiment, the method for identifying a compound capable of regulating the expression of a gene of the present invention provides a cell-based assay technique for identifying and evaluating agents which affect the expression of genes regulated by a class of Variable Number Tandem Repeat enhancer sequences. In this embodiment, an expression vector comprising a DNA sequence encoding a Variable Number Tandem Repeat, operatively linked to a reporter gene encoding an assayable product is introduced into a cell line and the recombinant cell line is cultured under conditions which permit expression of the assayable product. Therapeutic agents can then be identified by their ability to modulate the expression of the reporter gene, thereby affecting the production of the assayable product. Such agents are then tested for their ability to modulate the expression of the genes, preferably neuronal genes, with which the VNTR sequences are associated in their natural state.

A VNTR enhancer according to the present invention may be cloned from genomic DNA or may be synthesized de novo. Once inserted into an expression vector, the expression vector is introduced into an appropriate cell line. The recombinant cells are then cultured under conditions which permit expression of the reporter and result in formation of the assayable product.

After introduction of the expression vector, the cells are incubated with at least one compound to determine whether it possesses regulatory activity for expression of the reporter gene. Chemical agents and factors can be identified by their ability to modulate the expression of the reporter gene and thereby increase or decrease the production of the assayable product Such chemical compounds may be selected from chemical libraries, peptide libraries, collections of natural products and/or oligonucleotides, which may comprise nucleotide analogues.

### Therapeutic applications of the invention

Many genes associated with VNTR enhancers are associated with disease states in the human or animal body. For example, modification of the VNTR of the Huntington's (HD) gene is associated with Huntington's disease. In addition, other target genes having a VNTR sequence that are associated with human disorders include DRD4 and IL-1Ra (both of which are associated with attention deficit hyperactivity disorder), cytostatin B (which is implicated in myoclonus epilepsy) and the c-myc Ig fusion (implicated in Burkitt's lymphoma).

The present invention allows the identification of compounds for the selective stimulation or inhibition of expression of genes comprising enhancer sequences according to the present invention. These compounds may be used in the treatment of diseases associated with these genes.

Parkinson's Disease is associated with reduced levels of dopamine in the brain, and in particular with the loss of dopamineric neurons whose cell bodies are located in the substantia nigra. Treatment of this disorder has focused on dopamine agonists, such as L-dopa, which can restore dopamine levels in the brain, to a limited extent. In a preferred embodiment of the present invention, compounds are identified which are capable of selectively suppressing the expression of the human dopamine transporter gene. In this embodiment, an expression construct comprising the enhancer sequence from the human dopamine transporter gene is used to screen for compounds which decrease transcription of the reporter gene. The VNTR sequence located in the 3' untranslated region of the dopamine transporter gene has enhancer effects which increase the expression of the transporter, increase dopamine reuptake and thereby reduce dopaminergic neurotransmission. Selectively reducing the levels of the dopamine transporter may be an effective treatment for Parkinson's disease.

Schizophrenia is thought to be associated with raised levels of dopamine in the brain. A number of effective antipsychotic and neuroleptic drugs are thought to modulate or suppress dopaminergic neurotransmission. So, in contrast to Parkinson's disease, increasing levels of the dopamine transporter may be an effective treatment. Accordingly, in one embodiment of the present invention, compounds are identified which are capable of selectively promoting the expression of the human dopamine transporter gene. In this embodiment, an expression construct comprising the enhancer sequence from the human dopamine transporter gene is used to screen for compounds which increase transcription of the reporter gene.

Depression is a mental state that affects almost everyone, although for most the effects are short-lived, passing in a day or so. There are, however, those who suffer from chronic depression, which is an illness rather than a temporary mental state. People in this situation require medication in order to overcome their illness. The first drugs that were effective in the treatment of chronic depression were found to increase the levels of various amine neurotransmitters, including serotonin (5-hydroxytryptamine or 5HT) and dopamine. The mode of action of these drugs was either to prevent re-uptake of the neurotransmitters by transporter proteins in the cell surface membrane of transmitting cells or to prevent degradation of the neurotransmitters by the enzyme monoamine oxidase (MAO), The first drugs were relatively non-specific in their targets, affecting all of the amine-neurotransmitter transporter proteins. This causes a number of side-effects, such as low blood pressure due to changes in the amount norepinephrine (noradrenaline) released by nerves regulating heart rate. The earlier drugs were also found to affect acetylcholine receptors causing blurred vision, constipation and a dry mouth.

A new method by which re-uptake of dopamine may be inhibited has been discovered. Pharmaceutical intervention in this regulatory pathway will allow the levels of the dopamine transporter to be reduced. This pathway should be highly selective for dopamine re-uptake inhibition as it will reduce the overall level of the transporter protein available to take up dopamine. Moreover, as it is possible to screen for drugs that selectively modulate the enhancer activity of each genetic variant of the VNTR enhancer of the dopamine transporter gene, it will be possible to develop treatments that are chosen on the basis of the genetic makeup of the patient allowing for much more selective drugs. This will allow for more selective treatment of depression in those patients that respond to dopamine selective re-uptake inhibitors.

## Example 1

The following example describes DNA expression vectors comprising the VNTR sequence that is found in the human dopamine transporter gene, and the transcriptional enhancer and protein binding properties of this VNTR sequence. The example illustrates the construction of expression vectors for analysis of the VNTR sequence of the dopamine transporter using organotypic culture techniques.

Tandemly repeated sequences of many kinds are littered throughout the genomes of higher eukaryotes. Repeated sequences in eukaryotic genomes may be readily identified as they exhibit rapid renaturation kinetics when fragmented genomic DNA is denatured and then allowed to renature. Caesium Chloride gradient centrifugation of fragmented genomic DNA provides another method of identifying repetitive sequences as certain classes of repetitive sequence separate into discrete bands, that form 'satellites' to the largest band of DNA in the gradient. These satellite sequences tend to be found in heterochromatin at centromeric and telomeric locations in eukaryotic chromosomes. These heterochromatic sequences may have functional roles in chromosome structure. Another class of sequences that resemble satellites but which are typically not found in the satellite fractions of centrifugation gradients are 'microsatellite' sequences. These sequences typically comprise short tandemly repeated sequence ranging from short -CA-dinucleotide repeats to repeats of 10 or more base pairs. Certain classes of these sequences, such as -CA- dinucleotide repeats, are widely used in genetic fingerprinting and genome mapping because of the ease with which they are identified, the high number of these sequences in the genome and their relatively even distribution throughout the genome, particularly in 'gene-rich' euchromatic DNA. Many of these short, tandemly repeated sequences show variations in the number of tandem repeats in different individuals and these sequences are referred to as Variable Number Tandem Repeat (VNTR) sequences.

VNTR sequences are widely used in genetic studies as markers to identify different alleles of a gene. In most cases the VNTR sequence co-segregates with the allele of the gene of interest and variation in the VNTR sequence in the population means that different alleles of a nearby gene will often have a different VNTR associated with them. This allows the identity of a co-segregating allele to be determined by identification of the associated VNTR. Occasionally the VNTR sequence is biologically functional, in which case the variation in the marker is also the cause of the variation of phenotype of the associated gene. A number of VNTR sequences have been used as markers to identify alleles of the serotonin and dopamine transporter proteins, which are two medically important neuronal membrane proteins involved in the transport of monoamine neurotransmitters.

Monoamine transporters are a class of $Na^+/Cl^-$ dependent plasma membrane transporter proteins. The serotonin, noradrenaline and dopamine transporters are each encoded by a single gene and both regulate the termination of neurotransmission of their respective amine neurotransmitters by rapid reuptake of the transmitter. Various disorders result if too much or too little of these neurotransmitters are available during neurotransmission. Published results suggest that the transporter protein for dopamine is crucial in regulating the levels of these transmitters. (Jaber M., Jones S., Giros B. & Caron M.G., Mov Disord 12(5): 629-633, "The dopamine transporter: a crucial component regulating dopamine transmission." 1997). Similarly the serotonin transporter is important in regulating serotonin signalling.

There is an association between depression and the dopaminergic signalling system (Kapur S. & Mann J.J., Biol Psychiatry 32(1); 1-17, "Role of the dopaminergic system in depression." 1992). Decreased turnover of dopamine is believed to be strongly associated with certain forms of depression although the exact role of dopamine is still not completely clear. Lack of detailed knowledge notwithstanding, dopamine reuptake inhibitors, such as Nomifensine and alpha-amineptine, are known to be effective as anti-depressants and are particularly important in depressed patients with psychomotor retardation.

The dopamine transporter (DAT) is a plasma membrane transport protein that regulates dopamine-mediated (DA) neurotransmission by rapidly reaccumulating DA that has been released into the synapse. It is a member of a large family of sodium-and chloride-dependent transporters, including the closely related norepinephrine transporter and serotonin transporter (SERT) (Amara, S. G. and Sonders, M. S., 1998). DAT gene expression is limited exclusively to a subset of CNS (and retinal) DA neurons, yet robustly expressed within the midbrain (several hundred thousand DA neurons in humans). The human DAT gene (SLC6A3; also termed DAT1) is localized to chromosome 5p15.3 (Giros, B. et al., 1992; Vandenbergh, D. J. et al., 1992) and spans >60 kilobases. Within the 3' noncoding region of hDAT lies a 40 nucleotide repeat polymorphism, termed a variable number of tandem repeat (VNTR) polymorphism. Alleles of this 40 base pair VNTR sequence range from 3 to 13 repeats, with the 9-repeat and 10-repeat alleles by far the most common (Vandenbergh, D. J. et al., 1992). Some data suggests that the number of repeats in the VNTR is correlated with DAT ligand binding *in vivo* (Heinz, A. et al., 2000; Jacobsen, L. K. et al., 2000). A number of studies have investigated the possible association between this hDAT polymorphism and bipolar disorder, schizophrenia, Tourette's syndrome, drug abuse and drug-induced paranoia, alcoholism and alcohol withdrawal, and Parkinson's disease, with mixed results (Bannon et al. 1998; Vandenbergh, D. J. et al., 2000). However, the VNTR polymorphism has been consistently correlated with attention-deficit hyperactivity disorder (ADHD) (Barr, C. L. et al., 2001; Cook, E. H., Jr. et al., 1995; Gill, M. et al., 1997; Vandenbergh, D. J. et al., 2000; Waldman, I. D. et al., 1998).

The mechanism by which allelic differences in the VNTR region might affect hDAT function is unclear. Because the VNTR is in the 3' noncoding region, allelic variants cannot result in structural or functional differences in the hDAT protein. Recent studies have demonstrated that the SERT VNTR can function as an enhancer of gene transcription and, furthermore, that allelic variants of the SERT VNTR exhibit quantitatively and qualitatively different transcriptional activities (Fiskerstrand, C. E. et al., 1999; MacKenzie, A. and Quinn, J., 1999). In this study, we compare protein binding between DAT and SERT VNTR repeats and examine the ability of the hDAT VNTR to enhance transcription in dopaminergic cells.

*Electrophoretic mobility shift assays*

Electrophoretic mobility shift assay (EMSA) analysis was performed as described previously (Mendelson, S. C. and Quinn, J. P., 1995). Binding reactions were carried out in whole cell protein extracts prepared from ES cells. Each reaction contained 2.5$\mu$g poly d(I-C) and 0.5ng of $^{32}$P-3' end labelled double stranded oligonucleotide probe corresponding to one copy of repeat 3 of either DAT (5' CGTGTACTACCCCAGGACGCATGCAGGGCCCCCACTGGAG 3', SEQ ID NO:1) or the representative SERT VNTR element (GGCTGTGACCCGGGGTG, SEQ ID NO:4). 100ng non-specific oligonucleotide, NS (sense strand: 5' TCGAATCCCTTTAAATTTGCGAGC 3', SEQ ID NO:5) which binds the non-specific DNA binding complex Ku, is added to help reduce the intensity of a major non specific DNA binding complex often observed in EMSA (Quinn, J. P. and Farina, A. R., 1991). Increasing amounts (10, 50 or 100 ng) of competitor oligonucleotides were added as indicated.

*Construction of DAT VNTR reporter gene construct*

A 443-base pair fragment corresponding to the 9-repeat human DAT VNTR was amplified by PCR using the primers 5' TGTGGTGTAGGGAACGGCCTGAGAG 3' (SEQ ID NO:6) and 5' CCTTGAGCCGTGACCTCCAGGAA 3' (SEQ ID NO:7). This fragment was cloned into PCR 2.1 plasmid (Invitrogen). An enhancer-less vector (pGE3P) containing the SV40 viral promoter upstream of the green fluorescent protein (GFP) reporter gene was prepared from the pGL3P vector (Promega) by replacing the luciferase reporter gene with the GFP gene. The DAT VNTR fragment was excised from PCR 2.1 and subcloned upstream of the SV40 promoter into the pGE3P vector. The clone was sequenced to confirm element identity, orientation and insert copy number.

*Cell culture and lipid transfection of SN4741 cells*

SN4741 cells, a mouse embryonic substantia nigra derived cell line (Son, J. H. et al., 1999), were grown as previously described. Briefly, cells were grown at 33°C in 5 % $CO_2$ humidified atmosphere in DMEM high glucose (Gibco-BRL) containing 10 % heat-inactivated fetal calf serum (Hyclone) and supplemented with 0.6 % D-Glucose (Sigma), 1.4 mM L-glutamine, 10 U/ml penicillin and 10 mg/ml streptomycin as previously described (Sacchetti, P. et al., 2001). 1.5x10$^5$ cells were plated in 35 mM plates 24 hours prior to transfection. Three $\mu$g of hDAT VNTR GFP plasmid were complexed with 14.4 $\mu$L of LipofectAmine reagent (Gibco-BRL) and diluted in 1 mL of cell culture media. Cells were transfected with 1 $\mu$L the diluted DNA/lipid complex. After 5 hours of incubation, the DNA/lipid mix was replaced with fresh media and cells were incubated overnight. Cells were fixed with 4% paraformaldehyde, washed with phosphate-buffered saline (PBS) and viewed with a fluorescent microscope (Olympus).

*Biolistic transfection of organotypic slice cultures*

Animal use procedures were followed in strict accordance with the NIH *Guide to the Care and Use of Laboratory Animals* and a protocol approved by Wayne State University's Animal Investigation Committee. Organotypic cultures were prepared from rat midbrain slices as described by Stoppini et al., (1991) and Walker et al., (2000). Briefly, postnatal day 5 or 6 male Sprague Dawley rat pups were killed by decapitation. Coronal slices (300 $\mu$m) containing the substantia nigra were cut using a series 1000 vibratome (Technical Products, Inc.) and collected in oxygenated Ringer's buffer. Slices were washed three times with Minimum Essential Medium (MEM, Gibco BRL) and placed on 0.4 $\mu$m organotypic Millicell-CM inserts (Millipore). Slices were then transfected using a Helios Gene Gun with a modified "barrel"(Bio-Rad). DNA 'bullets' were prepared according to the manufacturer's instructions using 12.5 mg of 1 $\mu$m gold particles coated with 50 $\mu$g of DAT VNTR GFP plasmid. After transfection, slice cultures were incubated at 37°C with 5% $CO_2$ in culture

media (50% MEM (Gibco BRL), 25% Hank's solution (Gibco BRL), 25% heat-inactivated horse serum (Hyclone); 2mM L-glutamine (Hyclone); 2mM NaHCO$_3$ (Hyclone); 10 ng/ml glial-derived neurotrophic factor (Sigma). Forty-eight hours after transfection, slices were fixed in 4% paraformaldehyde and stored in 30% sucrose at 4°C until immunohistochemistry was performed.

*Immunohistochemistry*

Fixed slices were washed with PBS followed by one wash with 0.25% Triton X-100 in PBS (PBST). Slices were incubated with Block solution (0.25% Triton X-100; 5% normal goat serum; 5% normal donkey serum; 2% bovine serum albumin in PBS) for four hours at 4°C with gentle agitation, then incubated with a mouse monoclonal antibody to tyrosine hydroxylase in Block solution (1:2000) overnight at 4°C with gentle agitation. Slices were washed three times with PBST followed by a two hour incubation in Block solution at 18°C with goat anti-mouse IgG conjugated to cy3 (Jackson). After an additional three washes with PBST, slices were imaged using an Olympus Fluoview laser scanning confocal microscope equipped with argon and krypton lasers to detect GFP and cy3 fluorescence. The Olympus Fluoview software was also used to measure the quantity of green fluorescence in transfected cells. Optical densities of DAT VNTR GFP transfected cells were compared to the optical densities of cells transfected with the enhancer-less PGE3P GFP. One-tailed Student's t-tests were performed with Graph Pad Prism (Graph Pad Software).

*The VNTRs of the dopamine and serotonin transporters have overlapping protein binding properties*

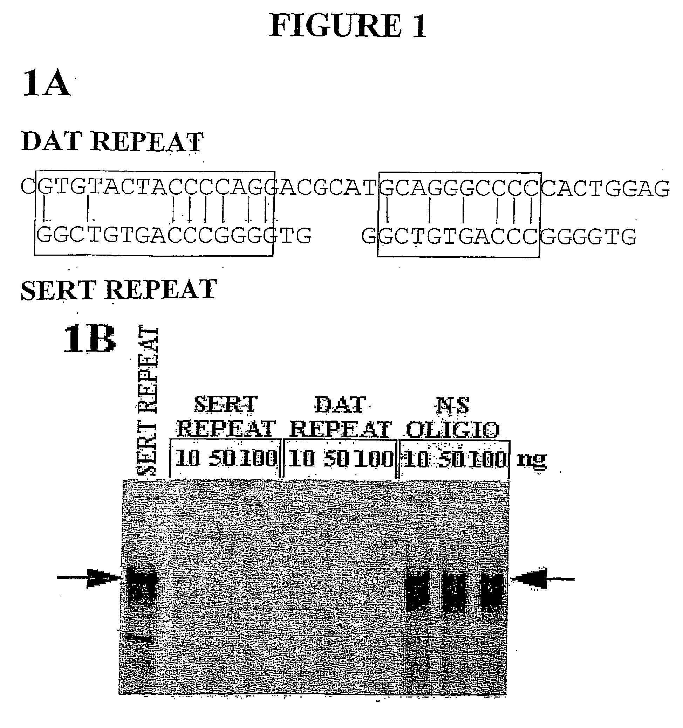

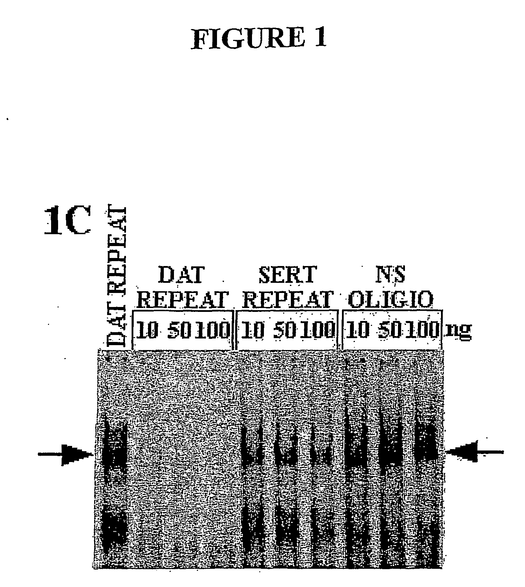

Alignment of a representative DAT and SERT VNTR repeat sequence is depicted in figure 1A. Each sequence corresponds to repeat three of the indicated VNTR. The highlighted region(s) depicts the maximum homology between the two sequences. The homology raises the possibility that these elements from the distinct VNTRs could be related in transcription factors that they bind and the element function. To test the hypothesis that both hDAT and SERT VNTR bind related transcription factors, we performed EMSA using oligonucleotides spanning the whole of the hDAT and SERT repeat number three. ES cells were used as they have been demonstrated to support expression of the SERT VNTR (Lovejoy et al) In figure 1B, EMSA analysis of embryonic stem (ES) cell protein binding to the SERT probe is shown in lane one. This binding is disrupted by increasing amounts of unlabeled SERT probe or by unlabeled hDAT VNTR probe, but not by the non-specific oligonucleotide. Similarly in figure 1C, ES cell protein binding to the hDAT VNTR probe was competed away by increasing concentrations of unlabeled hDAT VNTR probe and by unlabeled SERT probe, although the SERT probe was unable to completely compete off the labelled hDAT probe at the highest amount used. The inability of the SERT oligonucleotide to efficiently compete off the binding to the hDAT sequence may indicate additional transcription factor(s) binding to the hDAT VNTR sequence outside of the region with homology to the SERT VNTR.

*The hDAT VNTR is an enhancer in a neuronal cell line and neurons*

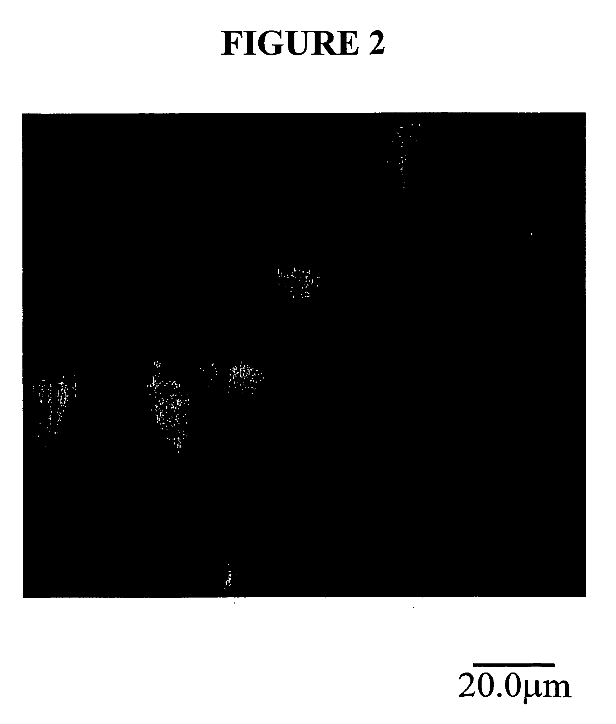

Given that the SERT VNTR has been shown to act as an enhancer *in vitro* and *in vivo* (Fiskerstrand, C. E. et al., 1999; MacKenzie, A. and Quinn, J., 1999), and that the SERT and hDAT VNTR sequences compete with each other for cell protein binding (figure 1), the hDAT VNTR may also act as an enhancer of gene transcription. To examine this possibility, we utilized an immortalized dopaminergic cell line. The SN4741 cell line derived from mouse substantia nigra does express DAT (Son, J. H. et al., 1999). SN4741 cells were transfected with a plasmid containing the hDAT VNTR cloned upstream of the minimal SV40 viral promoter driving expression of GFP. Other cells were transfected with the same promoter without the hDAT VNTR sequence. After 24 hours of incubation, cells expressing strong GFP fluorescence were visualized (Figure 2) suggesting that the hDAT VNTR sequence can indeed act as an enhancer. In contrast, no green fluorescence was observed in cells transfected with the enhancer-less control vector pGE3P (data not shown).

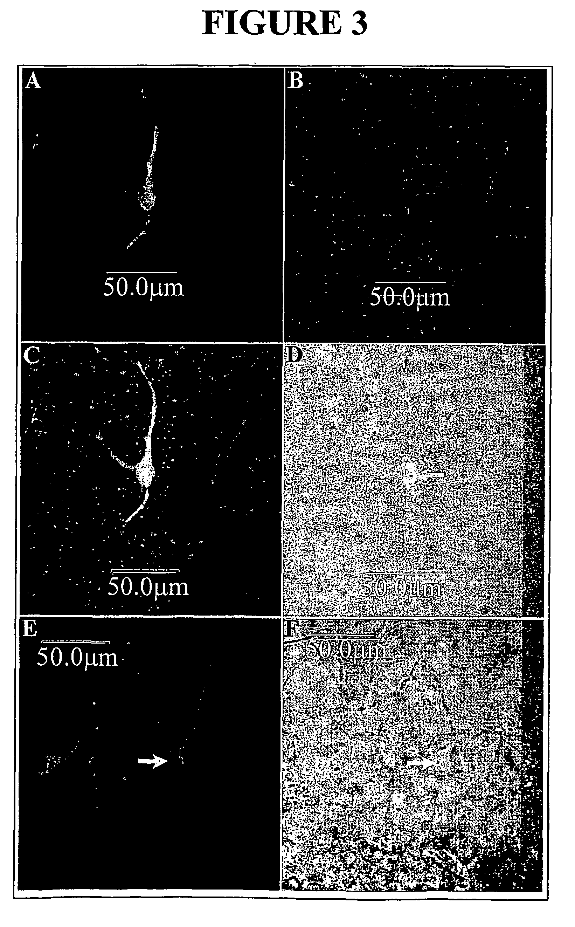

To further demonstrate that the DAT VNTR could act as a transcriptional enhancer, we utilized an organotypic midbrain slice culture method (Stoppini, L. et al., 1991; Walker, P. D. et al., 2000) and biolistic transfection to transfect primary dopaminergic neurons *in situ*. Organotypic midbrain slice cultures containing the substantia nigra were biolistically transfected with the hDAT VNTR GFP construct and incubated for forty-eight hours. To confirm that transfected cells (an example of which is shown in figure 3A) were dopaminergic, immunohistochemistry was performed with a monoclonal tyrosine hydroxylase antibody (figure 3B). The GFP signal co-localizes with the immunofluoresence as shown in the merged image where signal overlaps are depicted in yellow (figure 3C). The arrow in figure 3D indicates the gold bead that transfected this neuron. The enhancer-less control vector did not produce green fluorescence as seen in figure 3E, the computer merged image of the GFP signal and the cy3 immunofluoresence. Figure 3F contains the same cell as figure 3E, with the arrow indicating a gold bead in that cell.

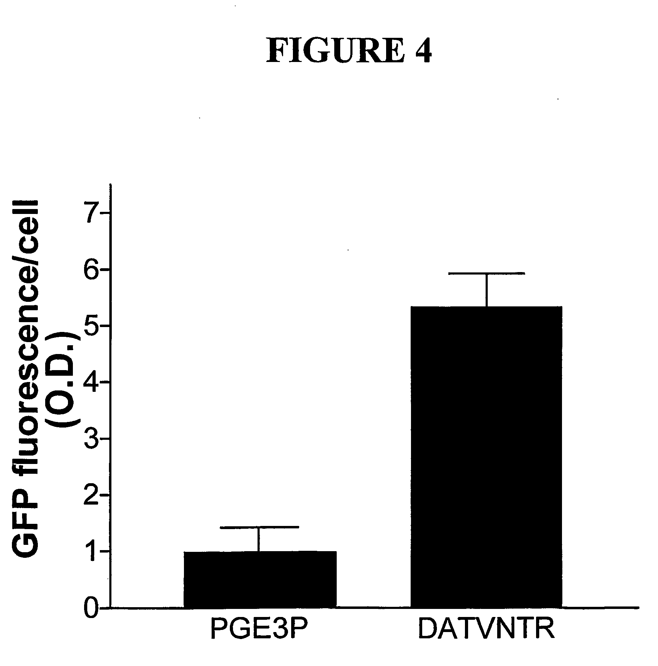

The average GFP fluorescence per cell in DAT VNTR transfected SN4741 cells was 5-fold higher than the fluorescence detected in cells transfected with the enhancer-less control vector pGE3p (figure 4) indicating that the hDAT VNTR was a potent transcriptional enhancer within SN4741 neurons.

The DAT has been implicated in many disorders thought to involve DA transmission and function. The focus of some of this research has been the DAT VNTR, which was identified when the human DAT gene was cloned (Giros, B. et al., 1992; Vandenbergh, D. J. et al., 1992). The DAT VNTR is located in the 3' noncoding region of the gene. While the number of repeats varies from three to eleven, the most common alleles contain 9- and 10-repeats of the VNTR (Barr, C. L. et al., 2001; Cook, E. H., Jr. et al., 1995; Gill, M. et al., 1997; Vandenbergh, D. J. et al., 2000; Waldman, I. D. et al., 1998). While studies of correlations of the DAT VNTR with many dopaminergic disorders and drug abuse are inconclusive (Bannon et al. 1998; Vandenbergh, D. J. et al., 2000), the association of the DAT VNTR with ADHD has been consistently observed (Barr, C. L. et al., 2001; Cook, E. H., Jr. et al., 1995; Gill, M. et al., 1997; Vandenbergh, D. J. et al., 2000; Waldman, I. D. et al., 1998).

Similarly to the DAT gene, the SERT gene also contains a VNTR, which is located in intron 2 (Lesch, K. P. et al., 1994). The SERT VNTR contains nine, ten or twelve repeats with the 10- and 12-repeat alleles being the most common (Ogilvie, A. D. et al., 1996). Many studies have examined the potential relationship between the SERT VNTR and mood disorders (Battersby, S. et al., 1996; Bellivier, F. et al., 1998; Collier, D. A. et al., 1996; Deary, I. J. et al., 1999; Gutierrez, B. et al., 1998; Gutierrez, B. et al., 1998; Kirov, G. et al., 1999; Ohara, K. et al., 1999; Rees, M. et al., 1997) and like the DAT VNTR, these studies have produced incongruous results.

Even though the VNTRs are in different regions of their respective genes, the sequences of individual repeats of the hDAT and SERT VNTR (an example of which is shown in fig 1A) have homologous regions. Given the resemblance between the hDAT and SERT VNTRs, it is possible that the same family of transcription factors would be able to alter transcription of the transporter genes. Our EMSA analysis (figures 1 B-C) supports this idea because the hDAT and SERT VNTR sequences did compete with each other for ES cell protein binding. While the hDAT oligo seemed to completely compete ES cell protein binding off the $^{32}$P-labelled SERT probe, the SERT oligo did reduce protein binding to the $^{32}$P-labelled hDAT probe. This may signify additional transcription factor(s) binding to the hDAT sequence outside of the homologous region, or that the hDAT repeat binds the putative factor(s) with higher affinity.

It was demonstrated previously that the SERT VNTR acts as a transcriptional enhancer (Fiskerstrand, C. E. et al., 1999; MacKenzie, A. and Quinn, J., 1999). Considering our evidence that the DAT VNTR is also a transcriptional enhancer *in vitro* and *ex vivo*, both of these VNTRs may be able to enhance transcription of their respective genes *in vivo*. This enhancement may depend upon the number of repeats found in the VNTR. In previous studies, the relative enhancing activity of the 10- and 12-repeat SERT VNTRs was compared and the 12-repeat demonstrated significantly stronger enhancement of transcription than the 10-repeat (Fiskerstrand, C. E. et al., 1999; MacKenzie, A. and Quinn, J., 1999). Humans homozygous for the 10-repeat DAT VNTR allele were found to have increased DAT binding compared to 9-/10-repeat heterozygotes (Heinz, A. et al., 2000). Conversely, Jacobsen, et al (2000), found a decrease in DAT binding in the 10-repeat homozygotic individuals. This decrease found by Jacobsen, et al (2000) in 10-repeat homozygotes might correlate with the poor response to methylphenidate found in 10-repeat homozygotes suffering from ADHD (Winsberg, B. G. and Comings, D. E., 1999) and a decrease in DAT binding found in patients suffering from depression (Laasonen-Balk, T. et al., 1999).

The SERT VNTR contains a Y-box element which can bind the transcription factor YB-1. The transcription factor may therefore modulate the expression of the SERT VNTR.

## Example 2

The following example describes DNA expression vectors comprising the VNTR sequence that is found in the human HD gene, and the transcriptional enhancer properties of this VNTR sequence.

*Construction of HD VNTR reporter gene construct*

A fragment corresponding to the 9 copies of the human HD VNTR was amplified by PCR was cloned into the pGL3P vector (Promega). The clone was sequenced to confirm element identity, orientation and insert copy number.

*Cell culture and lipid transfection of JAR cells*

JAR cells (available from ATCC) were grown at 37°C in 5 % $CO_2$ humidified atmosphere in DMEM (Gibco-BRL) containing 5 % fetal calf serum (Hyclone) and supplemented with 10 mM nonessential amino acids, 2 mM glutamine, 100 U/ml penicillin and 100 U/ml streptomycin. JAR cells were transfected with, pGL3, pGL3P and pGL3P containing the HD VNTR enhancer by electroporation according to the method described in Felgner et al. (Nucleic Acids Res. (1989) 15: 1311-1326).

*The HD VNTR is an enhancer in a platelet derived cell line*

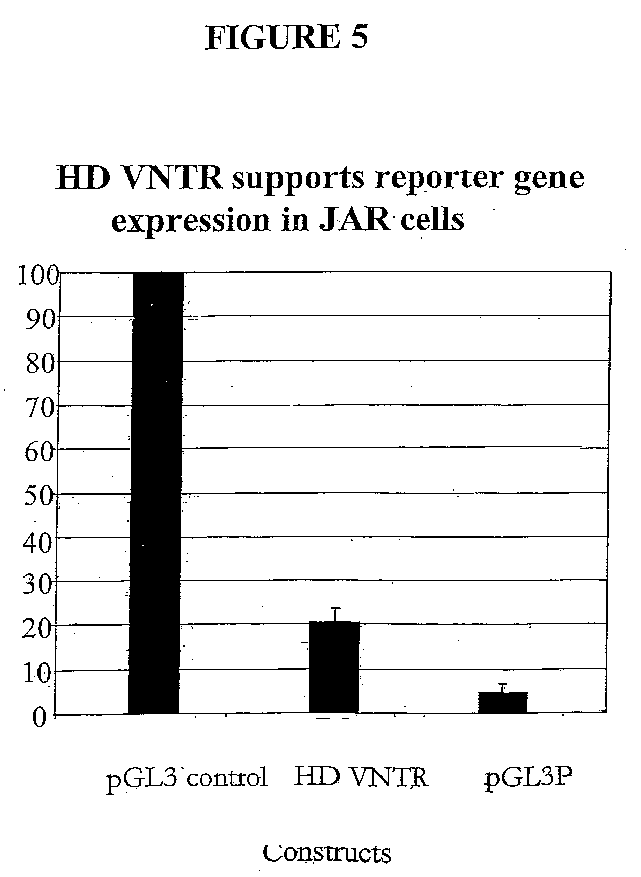

24 h after induction, the transfected cells were lysed with 1X lysis buffer according to the manufacturer's instructions (Promega). 20 μl of the lysate was then added to a luminometer tube containing 100 μl luciferase assay reagent (Promega). The luminescence was then read in a luminometer. The average luminescence of pGL3 (which contains a strong enhancer) was approximately 100 units. The average luminescence of cells transfected with pGL3P containing the HD VNTR enhancer was 20 units. The average luminescence of JAR cells transfected with the enhancer-less pGL3 plasmid was about 4 units. Therefore, there was a five fold increase in luminescence in cells transfected with a plasmid bearing the HD VNTR enhancer sequence (figure 5). Therefore, HD VNTR acts as a transcriptional enhancer within JAR cells.

REFERENCES

Amara S. G. and Sonders M. S. (1998) Neurotransmitter transporters as molecular targets for addictive drugs. Drug Alcohol Depend 51, 87-96.

Bannon M. J., Sacchetti P., and Granneman J. G. (1998) The dopamine transporter: potential involvement in neuropsychiatric disorders, in Psychopharmacology: The Fourth Generation of Progress (Watson S. J., ed.) On-line, http://www.acnp.org/G4/toc.htm.

Barr C. L., Xu C., Kroft J., Feng Y., Wigg K., Zai G., Tannock R., Schachar R., Malone M., Roberts W., Nothen M. M., Grunhage F., Vandenbergh D. J., Uhl G., Sunohara G., King N., and Kennedy J. L. (2001) Haplotype study of three polymorphisms at the dopamine transporter locus confirm linkage to attention-deficit/hyperactivity disorder. Biol Psychiatry 49, 333-339.

Battersby S., Ogilvie A. D., Smith C. A., Blackwood D. H., Muir W. J., Quinn J. P., Fink G., Goodwin G. M., and Harmar A. J. (1996) Structure of a variable number tandem repeat of the serotonin transporter gene and association with affective disorder. Psychiatr Genet 6, 177-181.

Bellivier F., Henry C., Szoke A., Schurhoff F., Nosten-Bertrand M., Feingold J., Launay J. M., Leboyer M., and Laplanche J. L. (1998) Serotonin transporter gene polymorphisms in patients with unipolar or bipolar depression. Neurosci Lett 255, 143-146.

Collier D. A., Stober G., Li T., Heils A., Catalano M., Di Bella D., Arranz M. J., Murray R. M., Vallada H. P., Bengel D., Muller C. R., Roberts G. W., Smeraldi E., Kirov G., Sham P., and Lesch K. P. (1996) A novel functional polymorphism within the promoter of the serotonin transporter gene: possible role in susceptibility to affective disorders. Mol Psychiatry 1, 453-460.

Cook E. H., Jr., Stein M. A., Krasowski M. D., Cox N. J., Olkon D. M., Kieffer J. E., and Leventhal B. L. (1995) Association of attention-deficit disorder and the dopamine transporter gene. Am J Hum Genet 56, 993-998.

Deary I. J., Battersby S., Whiteman M. C., Connor J. M., Fowkes F. G., and Harmar A. (1999) Neuroticism and polymorphisms in the serotonin transporter gene. Psychol Med 29, 735-739.

Felgner et al. (1989) Nucleic Acids Res. 15, 1311-1326.

Fiskerstrand C. E., Lovejoy E. A., and Quinn J. P. (1999) An intronic polymorphic domain often associated with susceptibility to affective disorders has allele dependent differential enhancer activity in embryonic stem cells. FEBS Lett 458, 171-174.

Gill M., Daly G., Heron S., Hawi Z., and Fitzgerald M. (1997) Confirmation of association between attention deficit hyperactivity disorder and a dopamine transporter polymorphism. Mol Psychiatry 2, 311-313.

Giros B., E1 Mestikawy S., Godinot N., Zheng K., Han H., Yang-Feng T., and Caron M. G. (1992) Cloning, pharmacological characterization, and chromosome assignment of the human dopamine transporter. Mol Pharmacol 42, 383-390.

Gutierrez B., Pintor L., Gasto C., Rosa A., Bertranpetit J., Vieta E., and Fananas L. (1998) Variability in the serotonin transporter gene and increased risk for major depression with melancholia. Hum Genet 103, 319-322.

Gutierrez B., Arranz M. J., Collier D. A., Valles V., Guillamat R., Bertranpetit J., Murray R. M., and Fanas L. (1998) Serotonin transporter gene and risk for bipolar affective disorder: an association study in Spanish population. Biol Psychiatry 43, 843-847.

Heinz A., Goldman D., Jones D. W., Palmour R., Hommer D., Gorey J. G., Lee K. S., Linnoila M., and Weinberger D. R. (2000) Genotype influences in vivo dopamine transporter availability in human striatum. Neuropsychopharmacology 22, 133-139.

Jacobsen L. K., Staley J. K., Zoghbi S. S., Seibyl J. P., Kosten T. R., Innis R. B., and Gelernter J. (2000) Prediction of dopamine transporter binding availability by genotype: a preliminary report. Am J Psychiatry 157, 1700-1703.

Kirov G., Rees M., Jones I., MacCandless F., Owen M. J., and Craddock N. (1999) Bipolar disorder and the serotonin transporter gene: a family-based association study. Psychol Med 29, 1249-1254.

Laasonen-Balk T., Kuikka J., Viinamaki H., Husso-Saastamoinen M., Lehtonen J., and Tiihonen J. (1999) Striatal dopamine transporter density in major depression. Psychopharmacology (Berl) 144, 282-285.

Lesch K. P., Balling U., Gross J., Strauss K., Wolozin B. L., Murphy D. L., and Riederer P. (1994) Organization of the human serotonin transporter gene. J Neural Transm Gen Sect 95, 157-162.

MacKenzie A. and Quinn J. (1999) A serotonin transporter gene intron 2 polymorphic region, correlated with affective disorders, has allele-dependent differential enhancer-like properties in the mouse embryo. Proc Natl Acad Sci USA 96, 15251-15255.

Mendelson S. C. and Quinn J. P. (1995) Characterisation of potential regulatory elements within the rat preprotachykinin-A promoter. Neurosci Lett 184, 125-128.

Mendelson S. C., Morrison C. F., McAllister J., Paterson J. M., Dobson S. P., Mulderry P. K., and Quinn J. P. (1995) Repression of preprotachykinin-A promoter activity is mediated by a proximal promoter element. Neuroscience 65, 837-847.

Nakamura Y., Koyama K., and Matsushima M. (1998) VNTR (variable number of tandem repeat) sequences as transcriptional, translational, or functional regulators. J Hum Genet 43, 149-152.

Ogilvie A. D., Battersby S., Bubb V. J., Fink G., Harmar A. J., Goodwim G. M., and Smith C. A. (1996) Polymorphism in serotonin transporter gene associated with susceptibility to major depression. Lancet 347, 731-733.

Ohara K., Suzuki Y., Ochiai M., Tsukamoto T., Tani K., and Ohara K. (1999) A variable-number-tandem-repeat of the serotonin transporter gene and anxiety disorders. Prog Neuropsychopharmacol Biol Psychiatry 23, 55-65.

Quinn J. P. and Farina A. R. (1991) Autoimmune antigen Ku is enriched on oligonucleotide columns distinct from those containing the octamer binding protein DNA consensus sequence. FEBS Lett 286, 225-228.

Rees M., Norton N., Jones I., McCandless F., Scourfield J., Holmans P., Moorhead S., Feldman E., Sadler S., Cole T., Redman K., Farmer A., McGuffin P., Owen M. J., and Craddock N. (1997) Association studies of bipolar disorder at the human serotonin transporter gene (hSERT; 5HTT). Mol Psychiatry 2, 398-402.

Sacchetti P., Mitchell T. R., Granneman J. G., and Bannon M. J. (2001) Nurr1 enhances transcription of the human dopamine transporter gene through a novel mechanism. J Neurochem 76, 1565-1572.

Son J. H., Chun H. S., Joh T. H., Cho S., Conti B., and Lee J. W. (1999) Neuroprotection and neuronal differentiation studies using substantia nigra dopaminergic cells derived from transgenic mouse embryos. J Neurosci 19, 10-20.

Stoppini L., Buchs P. A., and Muller D. (1991) A simple method for organotypic cultures of nervous tissue. J Neurosci Methods 37, 173-182.

Vandenbergh D. J., Persico A. M., Hawkins A. L., Griffin C. A., Li X., Jabs E. W., and Uhl G. R. (1992) Human dopamine transporter gene (DAT1) maps to chromosome 5p15.3 and displays a VNTR. Genomics 14, 1104-1106.

Vandenbergh D. J., Thompson M. D., Cook E. H., Bendahhou E., Nguyen T., Krasowski M. D., Zarrabian D., Comings D., Sellers E. M., Tyndale R. F., George S. R., O'Dowd B. F., and Uhl G. R. (2000) Human dopamine transporter gene: coding region conservation among normal, Tourette's disorder, alcohol dependence and attention-deficit hyperactivity disorder populations. Mol Psychiatry 5,283-292.

Waldman I. D., Rowe D. C., Abramowitz A., Kozel S. T., Mohr J. H., Sherman S. L., Cleveland H. H., Sanders M. L., Gard J. M., and Stever C. (1998) Association and linkage of the dopamine transporter gene and attention- deficit hyperactivity disorder in children: heterogeneity owing to diagnostic subtype and severity. Am J Hum Genet 63, 1767-1776.

Walker P. D., Andrade R., Quinn J. P., and Bannon M. J. (2000) Real-time analysis of preprotachykinin promoter activity in single cortical neurons. J Neurochem 75, 882-885.

Winsberg B. G. and Comings D. E. (1999) Association of the dopamine transporter gene (DAT1) with poor methylphenidate response. J Am Acad Child Adolesc Psychiatry 38, 1474-1477.

Kunugi H, Hattori M, Kato T et al. Serotonin transporter gene polymorphisms: ethnic difference and possible association with bipolar affective disorder. Mol Psychiatry. 1997;2:457-462

Furlong RA, Ho L, Walsh C et al. Analysis and meta-analysis of two serotonin transporter gene polymorphisms in bipolar and unipolar affective disorders. Am J Med Genet. 1998;81:58-63

Hoehe MR, Wendel B, Grunewald I et al. Serotonin transporter (5-HTT) gene polymorphisms are not associated with susceptibility to mood disorders. Am J Med Genet. 1998;81:1-3

**Claims**

1. An expression vector comprising:

      (a) an enhancer sequence; and
      (b) a reporter gene;

   wherein the enhancer sequence comprises:

      (i) the sequence of SEQ ID NO:1; or
      (ii) a sequence of at least 10 nucleotides which shows at least 80% sequence identity with a part of SEQ ID NO:1;

   and wherein the enhancer sequence is capable of promoting the transcription of the reporter gene.

2. An expression vector according to claim 1, wherein the enhancer sequence comprises the sequence of SEQ ID NO:1.

3. An expression vector according to claim 1 or claim 2, wherein the enhancer sequence comprises two or more repeats of a sequence as defined in (i) or (ii) of claim 1.

4. An expression vector according to claim 3, wherein the enhancer sequence comprises 9 or 10 repeats of SEQ ID NO:1.

5. An expression vector according to any preceding claim, wherein the reporter gene comprises a β-galactosidase gene.

6. A kit comprising two or more expression vectors, wherein each expression vector independently comprises:

      (a) an enhancer sequence; and
      (b) a reporter gene;

wherein the enhancer sequence comprises:

(i) the sequence of SEQ ID NO:1; or
(ii) a sequence of at least 10 nucleotides which shows at least 80% sequence identity with a part of SEQ ID NO:1;

and wherein the enhancer sequence is capable of promoting the transcription of the reporter gene.

7. A kit according to claim 6, wherein the enhancer sequence of each expression vector comprises the sequence of a different allele of a target gene found within the human population.

8. A kit according to claim 7, wherein the target gene is the human dopamine transporter gene.

9. A kit according to claim 8, wherein the enhancer sequence of at least one expression vector comprises 10 repeats of SEQ ID NO:1.

10. A cell comprising an expression vector as defined in any preceding claim.

11. A method for identifying a compound capable of regulating the expression of a target gene, wherein the target gene comprises an enhancer sequence as defined in any of claims 1 to 4 or 6 to 9, which method comprises:

(a) providing an expression vector as defined in any of claims 1 to 9;
(b) introducing the expression vector into a cell;
(c) contacting the cell with a compound; and
(d) detecting the expression of the reporter gene.

12. A method according to claim 11, wherein the target gene is the human dopamine transporter gene.

13. Use of an expression vector for identifying a compound capable of regulating the expression of a target gene, wherein the expression vector and the target gene each independently comprise an enhancer sequence as defined in any of claims 1 to 4 or 6 to 9.

14. Use according to claim 13, wherein the target gene is the human dopamine transporter gene.


**Patentansprüche**

1. Expressions-Vektor, umfassend:

(a) eine Verstärker-Sequenz und
(b) ein Reporter-Gen,

wobei die Verstärker-Sequenz umfasst:

(i) die Sequenz SEQ ID NO:1 oder
(ii) eine Sequenz von mindestens 10 Nucleotiden, die mindestens 80 % Sequenz-Identität mit einem Teil der SEQ ID NO:1 aufweisen,

und wobei die Verstärker-Sequenz in der Lage ist, die Transkription des Reporter-Gens zu fördern.

2. Expressions-Vektor nach Anspruch 1, wobei die Verstärker-Sequenz die Sequenz SEQ ID NO:1 umfasst.

3. Expressions-Vektor nach Anspruch 1 oder 2, wobei die Verstärker-Sequenz zwei oder mehr Wiederholungen einer in (i) oder (ii) des Anspruchs 1 definierten Sequenz umfasst.

4. Expressions-Vektor nach Anspruch 3, wobei die Verstärker-Sequenz 9 oder 10 Wiederholungen der SEQ ID NO: 1 umfasst.

5. Expressions-Vektor nach einem der vorangegangenen Ansprüche, wobei das Reporter-Gen ein Galactosidase-Gen umfasst.

6. Bausatz, umfassend zwei oder mehr Expressions-Vektoren,
   wobei jeder Expressions-Vektor unabhängig voneinander umfasst:

   (a) eine Verstärker-Sequenz und
   (b) ein Reporter-Gen,

   wobei die Verstärker-Sequenz umfasst:

   (i) die Sequenz SEQ ID NO:1 oder
   (ii) eine Sequenz von mindestens 10 Nucleotiden, die eine mindestens 80 % Sequenz-Identität mit einem Teil der SEQ ID NO:1 aufweisen,

   und wobei die Verstärker-Sequenz in der Lage ist, die Transkription des Reporter-Gens zu fördern.

7. Bausatz nach Anspruch 6, wobei die Verstärker-Sequenz eines jeden Expressions-Vektors die Sequenz eines unterschiedlichen Allels eines in der menschlichen Bevölkerung gefundenen Ziel-Gens umfasst.

8. Bausatz nach Anspruch 7, wobei das Ziel-Gen das menschliche Dopamin-Transporter-Gen ist.

9. Bausatz nach Anspruch 8, wobei die Verstärker-Sequenz mindestens eines Expressions-Vektors 10 Wiederholungen der SEQ ID NO:1 umfasst.

10. Zelle, umfassend einen Expressions-Vektor nach einem der vorangegangenen Ansprüche.

11. Verfahren zur Identifizierung einer Verbindung, die in der Lage ist, die Expression eine Ziel-Gens zu steuern, wobei das Ziel-Gen eine Verstärker-Sequenz nach einem der Ansprüche 1 bis 4 oder 6 bis 9 umfasst, wobei das Verfahren umfasst:

   (a) die Bereitstellung eines Expressions-Vektors nach einem der Ansprüche 1 bis 9,
   (b) die Einführung des Expressions-Vektors in eine Zelle,
   (c) das Zusammenbringen der Zelle mit einer Verbindung und
   (d) die Bestimmung der Expression des Reporter-Gens.

12. Verfahren nach Anspruch 11, wobei das Ziel-Gen das menschliche Dopamin-Transporter-Gen ist.

13. Verwendung eines Expressions-Vektors zur Identifizierung einer Verbindung, die in der Lage ist, die Expression eines Ziel-Gens zu steuern, wobei der Expressions-Vektor und das Ziel-Gen jeweils unabhängig voneinander eine Verstärker-Sequenz nach einem der Ansprüche 1 bis 4 oder 6 bis 9 umfassen.

14. Verwendung nach Anspruch 13, wobei das Ziel-Gen das menschliche Dopamin-Transporter-Gen ist.

**Revendications**

1. Vecteur d'expression comprenant :

   (a) une séquence d'activateur ; et
   (b) un gène rapporteur ;

   dans lequel la séquence d'activateur comprend :

   (i) la séquence de la SEQ ID N° 1 ; ou
   (ii) une séquence d'au moins 10 nucléotides qui présente une identité de séquence d'au moins 80 % avec une partie de la SEQ ID N° 1 ;

   et dans lequel la séquence d'activateur est capable de favoriser la transcription du gène rapporteur.

**2.** Vecteur d'expression suivant la revendication 1, dans lequel la séquence d'activateur comprend la séquence de la SEQ ID N° 1.

**3.** Vecteur d'expression suivant la revendication 1 ou la revendication 2, dans lequel la séquence d'activateur comprend deux ou plus de deux segments répétés d'une séquence telle que définie dans (i) ou (ii) de la revendication 1.

**4.** Vecteur d'expression suivant la revendication 3, dans lequel la séquence d'activateur comprend 9 ou 10 segments répétés de la SEQ ID N° 1.

**5.** Vecteur d'expression suivant l'une quelconque des revendications précédentes, dans lequel le gène rapporteur comprend un gène de β-galactosidase.

**6.** Kit comprenant deux ou plus de deux vecteurs d'expression, dans lequel chaque vecteur d'expression comprend indépendamment :

(a) une séquence d'activateur ; et
(b) un gène rapporteur ;

dans lequel la séquence d'activateur comprend :

(i) la séquence de la SEQ ID N° 1 ; ou
(ii) une séquence d'au moins 10 nucléotides qui présente une identité de séquence d'au moins 80 % avec une partie de la SEQ ID N° 1 ;

et dans lequel la séquence d'activateur est capable de favoriser la transcription du gène rapporteur.

**7.** Kit suivant la revendication 6, dans lequel la séquence d'activateur de chaque vecteur d'expression comprend la séquence d'un allèle différent d'un gène cible présent dans la population humaine.

**8.** Kit suivant la revendication 7, dans lequel le gène cible est le gène de transporteur de dopamine humain.

**9.** Kit suivant la revendication 8, dans lequel la séquence d'activateur d'au moins un vecteur d'expression comprend 10 segments répétés de la SEQ ID N° 1.

**10.** Cellule comprenant un vecteur d'expression tel que défini dans l'une quelconque des revendications précédentes.

**11.** Méthode pour identifier un composé capable de réguler l'expression d'un gène cible, dans laquelle le gène cible comprend une séquence d'activateur telle que définie dans l'une quelconque des revendications 1 à 4 ou 6 à 9, méthode qui comprend les étapes consistant à :

(a) fournir un vecteur d'expression tel que défini dans l'une quelconque des revendications 1 à 9 ;
(b) introduire le vecteur d'expression dans une cellule ;
(c) mettre en contact la cellule avec un composé ; et
(d) détecter l'expression du gène rapporteur.

**12.** Méthode suivant la revendication 11, dans laquelle le gène cible est le gène de transporteur de dopamine humain.

**13.** Utilisation d'un vecteur d'expression pour identifier un composé capable de réguler l'expression d'un gène cible, dans laquelle le vecteur d'expression et le gène cible comprennent chacun indépendamment une séquence d'activateur telle que définie dans l'une quelconque des revendications 1 à 4 ou 6 à 9.

**14.** Utilisation suivant la revendication 13, dans laquelle le gène cible est le gène de transporteur de dopamine humain.

# FIGURE 1

## 1A

**DAT REPEAT**

C GTGTACTACCCCAGG ACGCAT GCAGGGCCCC CACTGGAG

GGCTGTGACCCGGGG TG    G GCTGTGACCC GGGGTG

**SERT REPEAT**

## 1B

SERT REPEAT

| | SERT REPEAT | | | DAT REPEAT | | | NS OLIGIO | | | |
|---|---|---|---|---|---|---|---|---|---|---|
| | 10 | 50 | 100 | 10 | 50 | 100 | 10 | 50 | 100 | ng |

# FIGURE 1

# FIGURE 2

20.0μm

# FIGURE 3

# FIGURE 4

# FIGURE 5

## HD VNTR supports reporter gene expression in JAR cells

Constructs

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9113150 A

- WO 9739117 A

**Non-patent literature cited in the description**

- **FUKE, S. et al.** *Pharmacogenomics Journal,* 2001, vol. 1, 152-156
- **KIM S.H. et al.** Retroviral vectors. *Adv Virus Res,* 2000, vol. 55, 545-546
- **KURIAN K.M. et al.** Retroviral vectors. *Mol Pathol,* 2000, vol. 53 (4), 173-173
- **BENIHOUD K. ; YEH P. ; PERRICAUDET M.** *Curr Opin Biotechnol,* vol. 10 (5), 440-440
- *Adenovirus vectors for gene delivery,* 1999
- **RIDOUX V et al.** *Neurobiol Dis,* vol. 2 (1), 49-45
- *Adenovirus mediated gene transfer in organotypic brain slices,* 1995
- **KREMER E.J. ; PERRICAUDET M.** Adenovirus and adeno-associated virus mediated gene transfer. *Br Med Bull,* 1995, vol. 51 (1), 31-34
- **QUINN J.P. ; FISKERSTRAND C.E. ; GERRARD L. ; MACKENZIE A. ; PAYNE C.** Molecular models to analyse preprotachykinin-A expression and function. *Neuropeptides,* 2000, vol. 34, 292-302
- **FURTH P.A.** Gene transfer by biolistic process. *Mol Biotechnol,* 1997, vol. 7 (2), 139-143
- **SCHOEN P. et al.** Gene transfer mediated by fusion protein hemagglutinin reconstituted in cationic lipid vesicles. *Gene Ther,* 1999, vol. 6 (5), 823-823
- **ISAKA Y. et al.** The HVJ liposome method. *Exp Nephrol,* 1998, vol. 6 (2), 144-147
- **WU G.Y. et al.** Receptor-mediated gene delivery in vivo. Partial correction of genetic analbuminemia in Nagase rats. *J Biol Chem,* 1991, vol. 266 (22), 14338-14334
- **WAGNER E. ; OGRIS M. ; ZAUNER W.** Polylysine-based transfection systems utilizing receptor-mediated delivery. *Adv Drug Deliv Rev,* 1998, vol. 30, 97-113
- **ALTSCHUL, S.F. ; GISH, W. ; MILLER, W. ; MYERS, E.W. ; LIPMAN, D.J.** Basic local alignment search tool. *J. Mol. Biol.,* 1990, vol. 215, 403-410
- **SMITH ; WATERMAN.** *Journal of Molecular Biology,* 1981, vol. 147, 195-197
- **OLD ; PRIMROSE.** Principles of Gene Manipulation. Blackwell Science, 1994

- **MANIATIS et al.** Molecular Cloning, A Laboratory Manual. Cold Spring Harbor Laboratory, 1992
- **RYCHLIK W. ; SPENCER W.J. ; RHOADS R.E.** Optimization of the annealing temperature for DNA amplification in vitro. *Nucleic Acids Res,* 1990, vol. 18 (21), 6409-6412
- **LOWE T. et al.** A computer program for selection of oligonucleotide primers for polymerase chain reactions. *Nucleic Acids Res,* 1990, vol. 18 (7), 1757-1761
- **BRESLAUER K.J. et al.** Predicting DNA duplex stability from the base sequence. *Proc Nat1 Acad Sci U S A,* 1986, vol. 83 (11), 3746-3750
- **RYCHLIK W. ; RHOADS R.E.** A computer program for choosing optimal oligonucleotides for filter hybridization, sequencing and in vitro amplification of DNA. *Nucleic Acids Res,* 1989, vol. 17 (21), 8543-8551
- **WETMUR J.G.** DNA probes: applications of the principles of nucleic acid hybridization. *Crit Rev Biochem Mol Biol,* 1991, vol. 26 (3-4), 227-259
- **JACOBSEN L.K. et al.** Prediction of dopamine transporter binding availability by genotype: a preliminary report. *Am J Psychiatry,* 2000, vol. 157, 1700-1703
- **FISKERSTRAND C.E. ; LOVEJOY E.A. ; QUINN J.P.** An intronic polymorphic domain often associated with susceptibility to affective disorders has allele dependent differential enhancer activity in embryonic stem cells. *FEBS lett,* vol. 458, 171-174
- **MACKENZIE A. ; QUINN J.P.** A serotonin transporter gene intron 2 polymorphic region, correlated with affective disorders, has allele-dependent differential enhancer-like properties in the mouse embryo. *Proc Natl Acad Sci USA,* 1999, vol. 96, 15251-15255
- **JABER M. ; JONES S. ; GIROS B. ; CARON M.G.** The dopamine transporter: a crucial component regulating dopamine transmission. *Mov Disord,* 1997, vol. 12 (5), 629-633
- **FELGNER et al.** *Nucleic Acids Res.,* 1989, vol. 15, 1311-1326

- **AMARA S. G. ; SONDERS M. S.** Neurotransmitter transporters as molecular targets for addictive drugs. *Drug Alcohol Depend,* 1998, vol. 51, 87-96
- The dopamine transporter: potential involvement in neuropsychiatric disorders. **BANNON M. J. ; SACCHETTI P. ; GRANNEMAN J. G.** Psychopharmacology: The Fourth Generation of Progress. 1998
- **BARR C. L. ; XU C. ; KROFT J. ; FENG Y. ; WIGG K. ; ZAI G. ; TANNOCK R. ; SCHACHAR R. ; MALONE M. ; ROBERTS W.** Haplotype study of three polymorphisms at the dopamine transporter locus confirm linkage to attention-deficit/hyperactivity disorder. *Biol Psychiatry,* 2001, vol. 49, 333-339
- **BATTERSBY S. ; OGILVIE A. D. ; SMITH C. A. ; BLACKWOOD D. H. ; MUIR W. J. ; QUINN J. P. ; FINK G. ; GOODWIN G. M. ; HARMAR A. J.** Structure of a variable number tandem repeat of the serotonin transporter gene and association with affective disorder. *Psychiatr Genet,* 1996, vol. 6, 177-181
- **BELLIVIER F. ; HENRY C. ; SZOKE A. ; SCHURHOFF F. ; NOSTEN-BERTRAND M. ; FEINGOLD J. ; LAUNAY J. M. ; LEBOYER M. ; LAPLANCHE J. L.** Serotonin transporter gene polymorphisms in patients with unipolar or bipolar depression. *Neurosci Lett,* 1998, vol. 255, 143-146
- **COLLIER D. A. ; STOBER G. ; LI T. ; HEILS A. ; CATALANO M. ; DI BELLA D. ; ARRANZ M. J. ; MURRAY R. M. ; VALLADA H. P. ; BENGEL D.** A novel functional polymorphism within the promoter of the serotonin transporter gene: possible role in susceptibility to affective disorders. *Mol Psychiatry,* 1996, vol. 1, 453-460
- **COOK E. H., JR. ; STEIN M. A. ; KRASOWSKI M. D. ; COX N. J. ; OLKON D. M. ; KIEFFER J. E. ; LEVENTHAL B. L.** Association of attention-deficit disorder and the dopamine transporter gene. *Am J Hum Genet,* 1995, vol. 56, 993-998
- **DEARY I. J. ; BATTERSBY S. ; WHITEMAN M. C. ; CONNOR J. M. ; FOWKES F. G. ; HARMAR A.** Neuroticism and polymorphisms in the serotonin transporter gene. *Psychol Med,* 1999, vol. 29, 735-739
- **FELGNER et al.** *Nucleic Acids Res,* 1989, vol. 15, 1311-1326
- **FISKERSTRAND C. E. ; LOVEJOY E. A. ; QUINN J. P.** An intronic polymorphic domain often associated with susceptibility to affective disorders has allele dependent differential enhancer activity in embryonic stem cells. *FEBS Lett,* 1999, vol. 458, 171-174
- **GILL M. ; DALY G. ; HERON S. ; HAWI Z. ; FITZGERALD M.** Confirmation of association between attention deficit hyperactivity disorder and a dopamine transporter polymorphism. *Mol Psychiatry,* 1997, vol. 2, 311-313
- **GIROS B. ; E1 MESTIKAWY S. ; GODINOT N. ; ZHENG K. ; HAN H. ; YANG-FENG T. ; CARON M. G.** Cloning, pharmacological characterization, and chromosome assignment of the human dopamine transporter. *Mol Pharmacol,* 1992, vol. 42, 383-390
- **GUTIERREZ B. ; PINTOR L. ; GASTO C. ; ROSA A. ; BERTRANPETIT J. ; VIETA E. ; FANANAS L.** Variability in the serotonin transporter gene and increased risk for major depression with melancholia. *Hum Genet,* 1998, vol. 103, 319-322
- **GUTIERREZ B. ; ARRANZ M. J. ; COLLIER D. A. ; VALLES V. ; GUILLAMAT R. ; BERTRANPETIT J. ; MURRAY R. M. ; FANAS L.** Serotonin transporter gene and risk for bipolar affective disorder: an association study in Spanish population. *Biol Psychiatry,* 1998, vol. 43, 843-847
- **HEINZ A. ; GOLDMAN D. ; JONES D. W. ; PALMOUR R. ; HOMMER D. ; GOREY J. G. ; LEE K. S. ; LINNOILA M. ; WEINBERGER D. R.** Genotype influences in vivo dopamine transporter availability in human striatum. *Neuropsychopharmacology,* 2000, vol. 22, 133-139
- **JACOBSEN L. K. ; STALEY J. K. ; ZOGHBI S. S. ; SEIBYL J. P. ; KOSTEN T. R. ; INNIS R. B. ; GELERNTER J.** Prediction of dopamine transporter binding availability by genotype: a preliminary report. *Am J Psychiatry,* 2000, vol. 157, 1700-1703
- **KIROV G. ; REES M. ; JONES I. ; MACCANDLESS F. ; OWEN M. J. ; CRADDOCK N.** Bipolar disorder and the serotonin transporter gene: a family-based association study. *Psychol Med,* 1999, vol. 29, 1249-1254
- **LAASONEN-BALK T. ; KUIKKA J. ; VIINAMAKI H. ; HUSSO-SAASTAMOINEN M. ; LEHTONEN J. ; TIIHONEN J.** Striatal dopamine transporter density in major depression. *Psychopharmacology (Berl),* 1999, vol. 144, 282-285
- **LESCH K. P. ; BALLING U. ; GROSS J. ; STRAUSS K. ; WOLOZIN B. L. ; MURPHY D. L. ; RIEDERER P.** Organization of the human serotonin transporter gene. *J Neural Transm Gen Sect,* 1994, vol. 95, 157-162
- **MACKENZIE A. ; QUINN J.** A serotonin transporter gene intron 2 polymorphic region, correlated with affective disorders, has allele-dependent differential enhancer-like properties in the mouse embryo. *Proc Natl Acad Sci USA,* 1999, vol. 96, 15251-15255
- **MENDELSON S. C. ; QUINN J. P.** Characterisation of potential regulatory elements within the rat preprotachykinin-A promoter. *Neurosci Lett,* 1995, vol. 184, 125-128

- **MENDELSON S. C. ; MORRISON C. F. ; MCAL-LISTER J. ; PATERSON J. M. ; DOBSON S. P. ; MULDERRY P. K. ; QUINN J. P.** Repression of pre-protachykinin-A promoter activity is mediated by a proximal promoter element. *Neuroscience,* 1995, vol. 65, 837-847
- **NAKAMURA Y. ; KOYAMA K. ; MATSUSHIMA M.** VNTR (variable number of tandem repeat) sequences as transcriptional, translational, or functional regulators. *J Hum Genet,* 1998, vol. 43, 149-152
- **OGILVIE A. D. ; BATTERSBY S. ; BUBB V. J. ; FINK G. ; HARMAR A. J. ; GOODWIM G. M. ; SMITH C. A.** Polymorphism in serotonin transporter gene associated with susceptibility to major depression. *Lancet,* 1996, vol. 347, 731-733
- **OHARA K. ; SUZUKI Y. ; OCHIAI M. ; TSUKAMO-TO T. ; TANI K. ; OHARA K.** A variable-number-tandem-repeat of the serotonin transporter gene and anxiety disorders. *Prog Neuropsychopharmacol Biol Psychiatry,* 1999, vol. 23, 55-65
- **QUINN J. P. ; FARINA A. R.** Autoimmune antigen Ku is enriched on oligonucleotide columns distinct from those containing the octamer binding protein DNA consensus sequence. *FEBS Lett,* 1991, vol. 286, 225-228
- **REES M. ; NORTON N. ; JONES I. ; MCCAND-LESS F. ; SCOURFIELD J. ; HOLMANS P. ; MOORHEAD S. ; FELDMAN E. ; SADLER S. ; COLE T.** Association studies of bipolar disorder at the human serotonin transporter gene (hSERT; 5HTT. *Mol Psychiatry,* 1997, vol. 2, 398-402
- **SACCHETTI P. ; MITCHELL T. R. ; GRANNEMAN J. G. ; BANNON M. J.** Nurr1 enhances transcription of the human dopamine transporter gene through a novel mechanism. *J Neurochem,* 2001, vol. 76, 1565-1572
- **SON J. H. ; CHUN H. S. ; JOH T. H. ; CHO S. ; CON-TI B. ; LEE J. W.** Neuroprotection and neuronal differentiation studies using substantia nigra dopaminergic cells derived from transgenic mouse embryos. *J Neurosci,* 1999, vol. 19, 10-20
- **STOPPINI L. ; BUCHS P. A. ; MULLER D.** A simple method for organotypic cultures of nervous tissue. *J Neurosci Methods,* 1991, vol. 37, 173-182
- **VANDENBERGH D. J. ; PERSICO A. M. ; HAWKINS A. L. ; GRIFFIN C. A. ; LI X. ; JABS E. W. ; UHL G. R.** Human dopamine transporter gene (DAT1) maps to chromosome 5p15.3 and displays a VNTR. *Genomics,* 1992, vol. 14, 1104-1106
- **VANDENBERGH D. J. ; THOMPSON M. D. ; COOK E. H. ; BENDAHHOU E. ; NGUYEN T. ; KRA-SOWSKI M. D. ; ZARRABIAN D. ; COMINGS D. ; SELLERS E. M. ; TYNDALE R. F.** Human dopamine transporter gene: coding region conservation among normal, Tourette's disorder, alcohol dependence and attention-deficit hyperactivity disorder populations. *Mol Psychiatry,* 2000, vol. 5, 283-292
- **WALDMAN I. D. ; ROWE D. C. ; ABRAMOWITZ A. ; KOZEL S. T. ; MOHR J. H. ; SHERMAN S. L. ; CLEVELAND H. H. ; SANDERS M. L. ; GARD J. M. ; STEVER C.** Association and linkage of the dopamine transporter gene and attention- deficit hyperactivity disorder in children: heterogeneity owing to diagnostic subtype and severity. *Am J Hum Genet,* 1998, vol. 63, 1767-1776
- **WALKER P. D. ; ANDRADE R. ; QUINN J. P. ; BANNON M. J.** Real-time analysis of preprotachykinin promoter activity in single cortical neurons. *J Neurochem,* 2000, vol. 75, 882-885
- **WINSBERG B. G. ; COMINGS D. E.** Association of the dopamine transporter gene (DAT1) with poor methylphenidate response. *J Am Acad Child Adolesc Psychiatry,* 1999, vol. 38, 1474-1477
- **KUNUGI H ; HATTORI M ; KATO T et al.** Serotonin transporter gene polymorphisms: ethnic difference and possible association with bipolar affective disorder. *Mol Psychiatry,* 1997, vol. 2, 457-462
- **FURLONG RA ; HO L ; WALSH C et al.** Analysis and meta-analysis of two serotonin transporter gene polymorphisms in bipolar and unipolar affective disorders. *Am J Med Genet.,* 1998, vol. 81, 58-63
- **HOEHE MR ; WENDEL B ; GRUNEWALD I et al.** Serotonin transporter (5-HTT) gene polymorphisms are not associated with susceptibility to mood disorders. *Am J Med Genet,* 1998, vol. 81, 1-3